# EUROPÄISCHE PATENTSCHRIFT

(11) **EP 1 114 037 B1**
(45) Veröffentlichungstag und Bekanntmachung des Hinweises auf die Patenterteilung: **12.05.2004**
(21) Anmeldenummer: 99948841.4
(22) Anmeldetag: 16.09.1999
(51) Int. Cl.: C07D 265/36, C07D 265/34, C07D 279/16, C07D 413/12, A61K 31/538

(54) **BENZOXAZIN- UND BENZOTHIAZIN-DERIVATE UND DEREN VERWENDUNG IN ARZNEIMITTELN**
BENZOXAZINE AND BENZOTHIAZINE DERIVATIVES AND THEIR USE IN MEDICINES
DERIVES DE BENZOXAZINE ET DE BENZOTHIAZINE ET LEUR UTILISATION DANS DES PRODUITS PHARMACEUTIQUES

(30) Priorität: 18.09.1998 DE 19844291
(43) Veröffentlichungstag der Anmeldung: 11.07.2001
(73) Patentinhaber: Schering Aktiengesellschaft, 13353 Berlin (DE)
(72) Erfinder: HÖLSCHER, Peter, D-10559 Berlin (DE); REHWINKEL, Hartmut, D-12051 Berlin (DE); JAROCH, Stefan, D-10625 Berlin (DE); SÜLZLE, Detlev, D-10589 Berlin (DE); HILLMANN, Margrit, D-13437 Berlin (DE); BURTON, Gerardine, Anne, D-13591 Berlin (DE); McDONALD, Fiona, Mcdougall, D-14055 Berlin (DE)
(86) Internationale Anmeldenummer: PCT/EP1999/007089
(87) Internationale Veröffentlichungsnummer: WO 2000/017173

(56) Entgegenhaltungen:
- WO-A-97/45419
- WO-A-98/50372
- WO-A-99/12915
- DE-A- 2 509 155

## Beschreibung

Die Erfindung betrifft Benzoxazin- und Benzothiazin-Derivate, das Verfahren zu deren Herstellung und deren Verwendung in Arzneimitteln.

In menschlichen Zellen existieren mindestens 3 Formen von Stickstoffmonoxid-Synthasen, die Arginin in Stickstoffmonoxid (NO) und Citrullin überführen. Es wurden zwei konstitutive NO-Synthasen (NOS) identifiziert, die als Calzium / Calmodulin abhängige Enzyme im Gehirn (ncNOS oder NOS 1) bzw. im Endothel (ecNOS oder NOS 3) vorhanden sind. Eine weitere Isoform ist die induzierbare NOS (iNOS oder NOS 2), die ein praktisch Ca⁺⁺ unabhängiges Enzym ist und nach Aktivierung unterschiedlicher Zellen durch Endotoxin oder andere Stoffe induziert wird.

NOS-Inhibitoren und insbesondere selektive Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden. Eine Reihe von Reviews informiert über Wirkung und Inhibitoren von NO-Synthasen. Genannt seien beispielsweise: Drugs 1998, **1**, 321 oder Current Pharmac. Design 1997, **3**, 447.

Als NOS-Inhibitoren sind unterschiedliche Verbindungen bekannt. Beispielsweise werden Argininderivate, Aminopyridine, cyclische Amidinderivate, Phenylimidazole und andere beschrieben. Ähnlich substituierte Benzoxazinderivate sind aus WO 97 45419 und DE 25 09 155 A bekannt. Keiner Publikation ist zu entnehmen, daß 1,4-Benzoxazine und 1,4-Benzothiazine potent und selektiv Stickstoffmonoxid Synthasen inhibieren.

Es wurde nun gefunden, daß die erfindungsgemäß substituierten Heterocyclen gegenüber bekannten Verbindungen besonders vorteilhaft als Arzneimittel verwendet werden können.

Die Erfindung betrifft die Verbindungen der Formel I, deren tautomere und isomere Formen und Salze 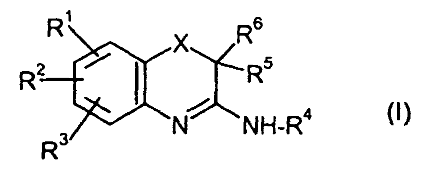 worin
- X: O, SOₘ oder Se,
- R¹: -(CHR⁹)ₙ-NR⁷-A-NR⁸-B
- R²: Wasserstoff ist oder
- R¹ und R²: gemeinsam mit zwei benachbarten Kohlenstoffatomen einen 5-, 6-, 7- oder 8gliedrigen Ring bilden, der monocyclisch oder bicyclisch, gesättigt oder ungesättigt ist und bei dem 1 oder 2 CH₂-Gruppen durch Sauerstoff oder Carbonyl ersetzt sein können und der mit -(CHR⁹)ᵣ-NR⁷-A-NR⁸-B substituiert ist und mit C₁₋₄-Alkyl substituiert sein kann,
- R³: Wasserstoff, Halogen, NO₂, Cyano, CF₃, -OCF₃, -S-R⁹, -O-R⁹, C₃₋₇-Cycloalkyl, -NR⁹-C(=NR¹⁰)-R¹¹, -NH-CS-NR¹²R¹³ , NH-CO-NR¹²R¹³, -SO₂NR¹²R¹³, -CO-NR¹²R¹³, -CO-R¹⁴, NR¹⁵R¹⁶, C₆₋₁₀Aryl, das gegebenenfalls mit Halogen, Cyano, C₁₋₄-Alkyl, -S-R⁹ oder -O-R⁹ substituiert ist,
5- oder 6gliedriges Heteroaryl mit 1 bis 4 Sauerstoff-, Schwefel- oder Stickstoffatomen
C₁₋₆-Alkyl, das gegebenenfalls mit Halogen, -OR⁹, -SR⁹, -NR¹²R¹³, =NR¹²,=NOC₁₋₆-Alkyl, =N-NHAryl, Phenyl, C₃₋₇-Cycloalkyl oder 5- oder 6gliedrigem Heteroaryl substituiert ist,
C₂₋₆-Alkenyl, das gegebenenfalls mit Halogen, CONH₂, C=N oder Phenyl substituiert ist,
C₂₋₆-Alkinyl, das gegebenenfalls mit Halogen, CONH₂, C≡N oder Phenyl substituiert ist.
- R⁴: Wasserstoff oder Acyl,
- R⁵ und R⁶: unabhängig voneinander Wasserstoff, C₃₋₇-Cycloalkyl, Phenyl, C₁₋₆-Alkyl, C₂₋₆-Alkenyl- oder C₂₋₆-Alkynylreste, die jeweils substituiert sein können mit Halogen, OH, O-C₁₋₆-Alkyl, SH, S-C₁₋₆-Alkyl, NR¹⁵R¹⁶, 5- oder 6gliedriges Heteroaryl mit 1 - 3 N-, O- oder S-Atomen, Phenyl oder C₃₋₇-Cycloalkyl,
- R⁷: Wasserstoff, C₁₋₆-Alkyl, das mit Phenyl substituiert sein kann, COOC₁₋₆-Alkyl oder CO-C₁₋₆-Alkyl,
- R⁸: Wasserstoff, C₁₋₆-Alkyl, das mit Phenyl substiuiert sein kann, COOC₁₋₆-alkyl oder COC₁₋₆-Alkyl,
- A: geradkettiges oder verzweigtes C₁₋₆-Alkylen, geradkettiges oder verzweigtes C₁₋₆-Alkenylen oder -(CH₂)ₚ-Q-(CH₂)_{q}-,
- B: Wasserstoff oder -(CH₂)ₚ-U,
- Q: C₃₋₇-Cycloalkyl, Indanyl, 5-, 6- oder 7gliedriges gesättigtes Heterocycloalkyl mit 1 - 2 N-, O- oder S-Atomen, C₆₋₁₀-Aryl oder 5- oder 6gliedriges Heteroaryl mit 1 - 3 N-, O- oder S-Atomen, das mit Benzol anelliert sein kann,
- U: Wasserstoff, gegebenenfalls mit Halogen substituiertes C₁₋₆-Alkyl, C₃₋₇-Cycloalkyl, Indanyl, C₇₋₁₀-Bicycloalkyl, C₆₋₁₀-Aryl oder 5- oder 6gliedriges Heteroaryl mit 1 - 3 N-, O- oder S-Atomen, das mit Benzol anelliert sein kann, wobei der Aryl- und Heteroarylrest substituiert sein kann mit Halogen, C₁₋₄-Alkyl, C₁₋₄-Alkoxy, CF₃, NO₂, NH₂, N(C₁₋₄-Alkyl)₂, Cyano, CONH₂, -O-CH₂-O-, -O-(CH₂)₂-O-, SO₂NH₂, OH, Phenoxy oder COOC₁₋₄-Alkyl,
oder
- R⁸ und B: gemeinsam mit dem Stickstoffatom einen 5 - 7gliedrigen gesättigten Heterocyclus bilden, der ein weiteres Sauerstoff-, Stickstoff- oder Schwefelatom enhalten kann und mit C₁₋₄-Alkyl, Phenyl, Benzyl oder Benzoyl substituiert sein kann oder einen ungesättigten 5gliedrigen Heterocyclus bilden, der 1 - 3 N-Atome enthalten und mit Phenyl C₁₋₄-Alkyl oder Halogen substituiert sein kann, oder
- R⁷ und A: gemeinsam mit dem Stickstoffatom einen 5 - 7gliedrigen gesättigten Heterocyclus bildet, der ein weiteres Sauerstoff-, Stickstoff- oder Schwefelatom enhalten kann oder einen ungesättigten 5gliedrigen Heterocyclus bildet, der 1 - 3 N-Atome enthalten kann,
- m: 0, 1 oder 2,
- n und r: 0, 1 bis 6,
- p und q: 0 bis 6 bedeuten,
- R⁹ und R¹⁰: Wasserstoff oder C₁₋₆-Alkyl,
- R¹¹: C₁₋₆-Alkyl, -NH₂, -NH-CH₃,-NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6gliedriges Heteroaryl mit 1 bis 4 Stickstoff-, Schwefel- oder Sauerstoffatomen,
- R¹² und R¹³: Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
- R¹⁴: Wasserstoff, Hydroxy, C₁₋₆-Alkoxy, Phenyl, gegebenenfalls mit CO₂H, CO₂C₁₋₆-Alkyl, Hydroxy, C₁₋₄-Alkoxy, Halogen, NR¹⁵R¹⁶,CONR¹²R¹³ oder Phenyl substituiertes C₁₋₆-Alkyl oder gegebenenfalls mit Phenyl, Cyano, CONR¹²R¹³ oder CO₂C₁₋₄-Alkyl substituiertes C₂₋₆-Alkenyl,
- R¹⁵ und R¹⁶: Wasserstoff, C₁₋₆-Alkyl, Phenyl oder Benzyl oder
- R¹⁵, R¹⁶: gemeinsam mit dem Stickstoffatom einen gesättigten 5-, 6- oder 7gliedrigen Ring bilden, der ein weiteres Stickstoff-, Sauerstoff- oder Schwefelatom enthalten und substituiert sein kann mit C₁₋₄-Alkyl, Phenyl, Benzyl oder Benzoyl
bedeuten, wobei,
falls X= O, R⁶ Methyl und R², R³, R⁴ und R⁵ Wasserstoff bedeuten, R¹ nicht 6-((4-Aminobenzyl)aminomethyl), 6-((4-Dimethylaminobenzyl)aminomethyl), 6-((4-Aminobenzyl)(tert.-butyloxycarbonyl)aminomethyl), 6-((4-Dimethylaminobenzyl) (tert.-butyloxycarbonyl)aminomethyl) ist.

Die Verbindungen der Formel können als Tautomere, Stereoisomere oder geometrische Isomere vorliegen. Die Erfindung umfaßt auch alle möglichen Isomeren wie E- und Z-Isomere, S- und R-Enantiomere, Diastereomere, Razemate und Gemische derselben einschließlich der tautomeren Verbindungen der Formel Ia und Ib

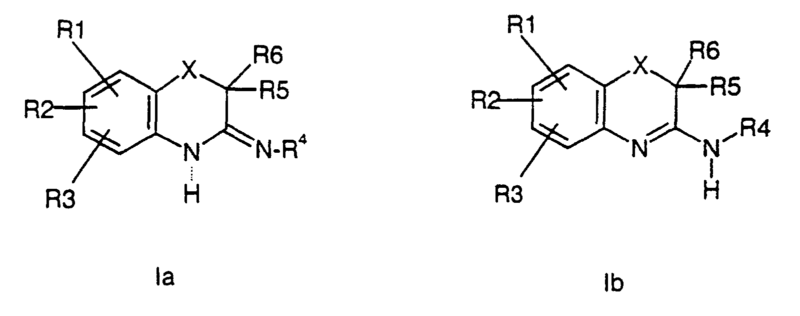

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, HCl, HBr, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek. Butyl, tert. Butyl, n-Pentyl, sek. Pentyl, tert. Pentyl, Neopentyl, n-Hexyl, sek. Hexyl, Heptyl, Octyl.

Ist der Alkylrest U mit Halogen substituiert, so kann er ein- bis mehrfach halogeniert und perhalogeniert sein wie beispielsweise Trifluormethyl, Trifluorethyl.

Alkenyl- und Alkynyl-Substituenten enthalten vorzugsweise eine Doppelbindung und sind jeweils geradkettig oder verzweigt. Beispielsweise seien die folgenden Reste genannt: Vinyl, 2-Propenyl, 1-Propenyl, 2-Butenyl, 1-Butenyl, 3-Butenyl, 2-Methyl-2-propenyl, 2-Pentenyl, 4-Hexenyl, Ethinyl, 1-Propinyl, 2-Propinyl, 1-Butinyl, 2-Butinyl.

Unter Cycloalkyl ist jeweils Cyclopropyl, Cyclobutyl, Cyclopentyl, Cyclohexyl oder Cycloheptyl zu verstehen. Als Bicyclus seien beispielsweise Bicycloheptan und Bicyclooctan genannt.

Halogen bedeutet jeweils Fluor, Chlor, Brom oder Jo.d.

Unter Aryl ist jeweils Naphthyl oder Phenyl zu verstehen, die ein- bis dreifach gleich oder verschieden substituiert sein können.

Als Heteroarylreste, die über das Heteroatom oder ein Kohlenstoffatom gebunden sein können, seien beispielsweise die folgenden 5- und 6-Ringheteroaromaten genannt:
Imidazol, Indol, Isooxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin. Chinolin. Als Heteroarylrest ist auch 2-C₁₋₆-Alkyl-3-amino-1,4-benzoxazin und 2-C₁₋₆-Alkyl-3-keto-1,4-benzoxazin geeignet.

Als bevorzugte Ausführungsform für R¹¹ in der Bedeutung Heteroaryl ist Thienyl zu betrachten.

Als gesättigter Heterocyclus sei beispielsweise Piperidin, Pyrrolidin, Morpholin, Thiomorpholin, Hexahydroazepin und Piperazin genannt. Der Heterocyclus kann 1 - 3fach substituiert sein mit C₁₋₄-Alkyl oder einem gegebenenfalls mit Halogen substituierten Phenyl-, Benzyl- oder Benzoylrest. Beispielsweise seien genannt: N-Methyl-piperazin, 2,6-Dimethylmorpholin, Phenylpiperazin oder 4-(4-Fluorbenzoyl)-piperidin.

Bilden -NR⁸B oder -NR⁷-A- gemeinsam mit dem Stickstoffatom einen ungesättigten Heterocyclus, so seien beispielsweise imidazol, Pyrrol, Pyrazol und Triazol genannt.

Für die Substituenten R⁵ und R⁶ in Position 2 des Oxazins oder Thiazins ist einfache Substitution bevorzugt, wobei der Substituent R⁶ insbesondere C₁₋₆-Alkyl und der Substituent R⁵ insbesondere Wasserstoff bedeutet.

Der Substituent Q kann an beliebiger Stelle über ein C-Atom oder gegebenenfalls über ein N-Atom verknüpft sein.

Bilden R¹ und R² gemeinsam mit zwei benachbarten Kohlenstoffatomen einen Ring, so kann dieser in Position 5, 6 oder 6, 7 oder 7, 8 des Benzoxazins bzw. Benzothiazins stehen und hat die Formel 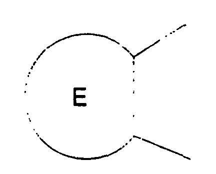 worin
E einen gesättigten oder ungesättigten C₃₋₈-Alkylenrest bedeutet, der 1 bis 2-fach mit -(CHR⁹)ᵣ-NR⁷-A-NR⁸B und gegebenenfalls 1 - 2fach mit C₁₋₄-Alkyl substituiert ist und bei dem 1 oder 2 CH₂-Gruppen durch Sauerstoff, Carbonyl oder dessen Derivat ersetzt sein können, wobei der Alkylenrest einen ankondensierten Benzolrest enthalten kann wie beispielsweise Indan oder als Bicyclus vorliegen kann wie beispielsweise Bicycloheptan.

Als Strukturen von E seien beispielsweise genannt:

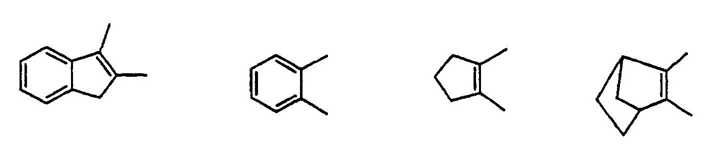

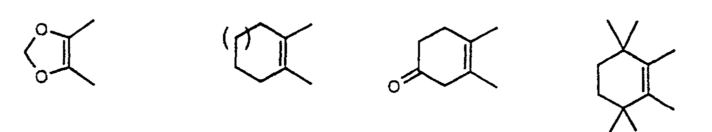

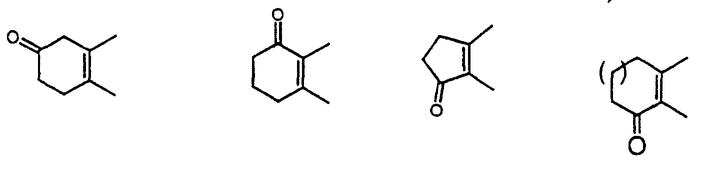

Als Carbonylderivate sind beispielsweise =NOH, =N-OC₁₋₆-Alkyl, =NH-NH₂, =N-NH-Phenyl geeignet.

Vorzugsweise sind zwei benachbarte Kohlenstoffatome des Aromaten mit C₁₋₆-Alkylen zu einem 3 - 8gliedrigen, insbesondere 5 - 6gliedrigen ungesättigten Ring verknüpft, der in beliebiger Position substituiert ist, insbesondere bedeutet E gesättigtes oder ungesättigtes C₅₋₆-Alkylen, das mit -(CHR⁹),-NR⁷-A-NR⁸B substituiert ist, wobei r insbesondere null bedeutet.

Der Acylrest R⁴ leitet sich von geradkettigen oder verzweigten aliphatischen Carbonsäuren ab wie beispielsweise Ameisensäure, Essigsäure, Propionsäure, Buttersäure, Trimethylessigsäure oder Capronsäure oder von bekannten Benzolsulfonsäuren, die mit Halogen oder C₁₋₄-Alkyl substituiert sein können, sowie C₁₋₄-Alkansulfonsäuren wie beispielsweise Methansulfonsäure, p-Toluolsulfonsäure.

Bevorzugte Ausführungsformen von X sind S und O.

R⁴, R⁷ und R⁸ bedeuten jeweils vorzugsweise Wasserstoff und eine bevorzugte Ausführungsform von R³ ist Wasserstoff.

Die Bedeutung von n ist vorzugsweise ungleich null.

Die Substituenten R⁷ und R⁸ bedeuten vorzugsweise Wasserstoff.

Eine bevorzugte Ausführungsform von A ist insbesondere geradkettiges oder verzweigtes C₁₋₆-Alkylen oder -(CH₂)ₚ-Q-(CH₂)_{q}-, wobei p und q jeweils insbesondere 1 - 4 bedeuten.

Bevorzugte Ausführungsformen von U sind Wasserstoff, gegebenenfalls mit Halogen substituiertes C₁₋₆-Alkyl, C₃₋₇-Cycloalkyl und Phenyl, das mit Halogen, C₁₋₄-Alkyl, C₁₋₄-Alkoxy, CF₃, NO₂, NH₂, N(C₁₋₄-Alkyl)₂, Cyano, CONH₂, -O-CH₂-O-, -O-(CH₂)₂-O-, SO₂NH₂, OH, Phenoxy oder COOC₁₋₄-Alkyl substituiert sein kann.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

### Beispielsweise seien genannt:

Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korksakoff's Disease, Epilepsie, Erbrechen, Schlafstörungen, Schizophrenie, Depression, Stress, Schmerz, Migräne, Hypoglykämie, Demenz wie z.B. Alzheimersche Krankheit, HIV-Demenz und Presenile Demenz.

Ferner eignen sie sich zur Behandlung von Krankheiten des Herz-Kreislauf Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens/Darms (bowel disease), von Meningitis, Glomerulonephritis, akute und chronische Lebererkrankungen, Erkrankungen durch Abstoßung (beispielsweise allogene Herz-, Nieren- oder Lebertransplantationen) oder entzündlichen Hautkrankheiten wie Psoriasis und andere.

Auf Grund ihres Wirkprofils eignen sich die erfindungsgemäßen Verbindungen sehr gut zur Inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfs- und/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch in Form von Aerosolen oder transdermalen Systemen oder intrathekal erfolgen. Als Hilfsstoffe für die gewünschte Arzneimittelformulierung sind die dem Fachmann bekannten inerten organischen und anorganischen Trägermaterialien geeignet wie z.B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1 - 2000 mg, vorzugsweise 20 - 500 mg, wobei die Dosis als einmal zu verabreichende Einzeldosis oder unterteilt in zwei oder mehreren Tagesdosen gegeben werden kann.

Die NOS-inhibitorische Wirksamkeit der Verbindungen der Formel I und deren physiologisch verträglicher Salze kann nach den Methoden von Bredt und Snyder in Proc. Natl. Acad. Sci. USA (1989) 86, 9030-9033 bestimmt werden.

Die Herstellung der erfindungsgemäßen Verbindungen erfolgt dadurch, daß man eine Verbindung der Formel II oder deren Salz 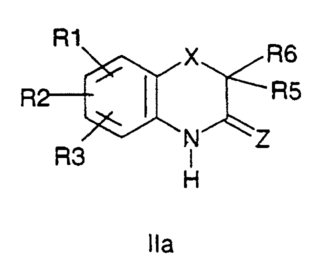 oder 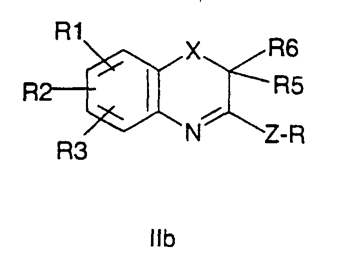 worin R¹, R², R³, R⁵, R⁶ und X die obige Bedeutung haben, Z Sauerstoff oder Schwefel ist und R C₁₋₆-Alkyl bedeutet, mit Ammoniak oder primären Aminen umsetzt, wobei vorhandene Aminogruppen gegebenenfalls intermediär geschützt sind, und gewünschtenfalls anschließend acyliert, die Isomeren trennt oder die Salze bildet.

Die Umsetzung mit Ammoniak gelingt unter Druck in Autoklaven bei Ammoniaküberschuß bei tiefen Temperaturen (-78 °C) oder durch Rühren in mit Ammoniak gesättigten Methanol bei Raumtemperatur. Bevorzugt werden Thiolactame umgesetzt. Wird mit Aminen umgesetzt, so stellt man aus dem Lactam oder Thiolactam zunächst den Iminether oder Iminothioether als Zwischenverbindung dar (z.B. mit Methyliodid oder Methylsulfat) und setzt diesen mit oder ohne Isolierung der Zwischenverbindung mit den entsprechenden Aminen oder deren Salzen um.

Als Aminoschutzgruppen sind beispielsweise Carbamate wie tert. Butoxycarbonyl, Benzyloxycarbonyl oder Acetyl geeignet.

An den Vorstufen werden gewünschtenfalls Sulfide oxidiert, Ester verseift, Säuren verestert, Hydroxygruppen verethert oder acyliert, Amine acyliert, alkyliert, diazotiert, halogeniert, NO₂ eingeführt oder reduziert, mit Isocyanaten oder Isothiocyanaten umgesetzt, die Isomeren getrennt oder die Salze gebildet.

Die Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und ggf. Salze der Aminobenzoxazine oder -thiazine weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Die Reduktion einer Estergruppe zum Alkohol erfolgt in an sich bekannter Weise mit DiBAH in geeignetem Lösungsmittel bei tiefen Temperaturen. Die reduktive Aminierung eines Ketons oder eines Benzaldehyds mit Amin unter Zugabe eines Borhydrides gibt benzylische Amine. Mit passend gewählten Diaminen erhält man nach Zugabe von gleichen oder verschiedenen Aldehyden symmetrische oder unsymmetrische Aminoverbindungen.

Zusätzlich kann durch elektrophile aromatische Substitution eine Nitrogruppe oder Halogen, insbesondere Brom, eingeführt werden. Dabei entstehende Gemische können in üblicher Weise, auch mittels HPLC, getrennt werden. Wenn ein Nitril vorliegt, kann dieses nach bekannten Verfahren verseift werden oder in das entsprechende Amin, Tetrazol oder Amidoxim überführt werden, oder es wird durch Angriff von substituierten Anilinen oder Aminen zu einem substituierten Amidin.

Die Friedel-Crafts Acylierung wird bei Lactamen vom Typ IIa erfolgreich angewandt, und anschliessend kann selektiv das Lactam in das Thiolactam überführt oder das Acylierungsprodukt reduktiv aminiert werden.

Die Reduktion der Nitrogruppe oder ggf. der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat oder Ameisensäure in bekannter Weise benutzt werden. Reduktionsmittel wie Zinn-IIchlorid können ebenso verwendet werden wie komplexe Metallhydride eventuell in Gegenwart von Schwermetallsalzen. Es kann vorteilhaft sein, vor der Reduktion die Estergruppe wie in Formel V einzuführen. Für Nitrogruppen bewährt hat sich die Reduktion mit Zink oder Eisen in Essigsäure.

Wird eine einfache oder mehrfache Alkylierung einer Aminogruppe oder einer CH-aciden Kohlenstoffposition gewünscht, so kann nach üblichen Methoden beispielsweise mit Alkylhalogeniden alkyliert werden.Gegebenenfalls ist Schutz der Lactamgruppe als Anion durch ein 2. Equivalent Base oder durch eine passende Schutzgruppe erforderlich.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt. Benzylalkohole lassen sich wie üblich mit Methansulfonylchlorid in die entsprechenden Benzylhalogenide überführen.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser oder konz. Schwefelsäure als Lösungsmittel bei Temperaturen zwischen -10 °C und 30 °C.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch Chromatographie an chiralen Phasen sowie durch stereoselektive Synthese erhalten werden.

Die Herstellung der Salze erfolgt in üblicher Weise, indem man eine Lösung der Verbindung der Formel I - gegebenenfalls auch mit geschützten Aminogruppen - mit der äquivalenten Menge oder einem Überschuß einer Säure, die gegebenenfalls in Lösung ist, versetzt und den Niederschlag abtrennt oder in üblicher Weise die Lösung aufarbeitet.

Nucleophile Substitution von Benzylhalogeniden mit sekundären Aminen liefert die korrespondierenden Benzylamine.

Thiolactame der Formel IIa (Z = S) erhält man beispielsweise aus Lactamen mit Phosphorpentasulfid (P₄S₁₀) oder Lawessons Reagenz (2,4-Bis(4-methoxphenyl)-1,3,2,4-dithiaphosphetan-2,4-disulfid) in geeigneten Lösungsmitteln und Verbindungen der Formell IIb können beispielsweise durch Umsetzung mit Meerwein-Reagenz (Trimethyloxoniumtetrafluoroborat) erhalten werden.

Soweit die Herstellung der Ausgangsverbindungen nicht beschrieben wird, sind diese bekannt und käuflich oder analog zu bekannten Verbindungen oder nach hier beschriebenen Verfahren herstellbar.

Die Erfindung betrifft auch die Zwischenverbindungen der Formel IIa und IIb und deren Salze 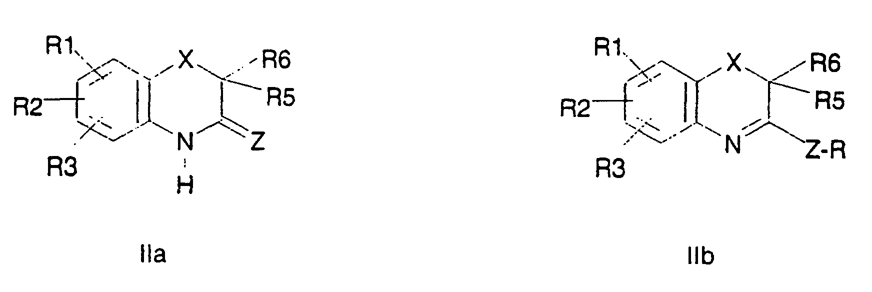 worin

R¹, R², R³, R⁵, R⁶ und X die obige Bedeutung haben, Z Sauerstoff oder Schwefel ist und R C₁₋₆-Alkyl bedeutet.

Die Herstellung der Verbindungen der Forml IIa kann beispielsweise dadurch erfolgen, daß man eine Verbindung der Formel III 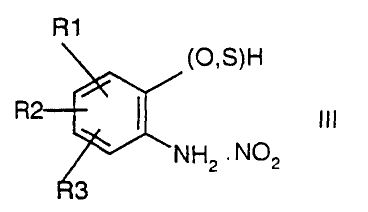 worin R¹ bis R³ die obige Bedeutung haben mit einer Verbindung der Formel IV 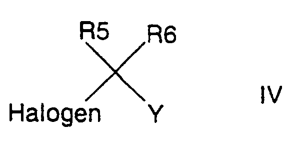 worin R⁵ und R⁶ die obige Bedeutung haben und Y eine reaktive Carboxylgruppe ist wie Säurehalogenid, Nitril, Carbonsäureester umsetzt und gegebenenfalls reduktiv cyclisiert oder dadurch, daß man eine Verbindung der Formel V 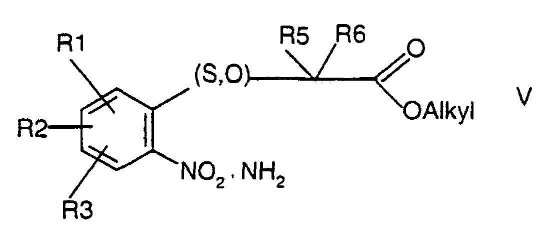 reduktiv cyclisiert.

Aromatische Thiole vom Typ III erhält man unter anderem wie in Chem. Pharm. Bull. 1991, **39**, 2888 und der dort genannten Literatur beschrieben durch Umlagerung der entsprechenden Dimethylaminothiocarbamate.

Die Einführung der Substituenten R¹ - R³ kann auf der Stufe der Verbindungen der Formel III oder II erfolgen.

Zur Herstellung von Verbindungen der Formel II kann der Aldehyd oder das Keton des entsprechenden 1,4-Benzoxazin-3-ons bzw. 1,4-Benzothiazin-3-ons reduktiv aminiert werden. Dies gelingt auch zweifach mit passend gewählten Diaminen. Diamine lassen sich auch umsetzen mit dem Aldehyd des 1,4-Benzoxazin-3-ons sowie gleichzeitig mit passend gewählten anderen Aldehyden. Wird die Einführung eines Heteroarylrestes Q gewünscht, so kann das entsprechende Halogenderivat nucleophil substituiert werden. Ist eine primäre oder sekundäre Aminogruppe vorhanden, so kann es vorteilhaft sein, diese intermediär zu schützen, beispielsweise durch Einführung einer tert. Butoxycarbonylgruppe, die nach der Amidin-Bildung in üblicher Weise abgespalten wird. Die Herstellung pharmakologisch wirksamer Verbindungen aus den Zwischenprodukten erfolgt wie vorn beschrieben.

Neue Verbindungen wurden durch eine oder mehrere der folgenden Methoden charakterisiert: Schmelzpunkt, Massenspektroskopie, Infrarotspektroskopie, Nuklearmagnetische Resonanzspektroskopie (NMR). NMR-Spektren wurden mit einem Bruker 300 MHz Gerät gemessen, die (deuterierten) Lösemittel werden jeweils angegeben und wie folgt abgekürzt: CDCl₃ (Chloroform), DMSO (Dimethylsulfoxid). Verschiebungen sind in delta und ppm angegeben. Es bedeuten: m (Multiplett, mehrere Signale), s (Singulett), d (Dublett), dd (Doppeldublett usw.), tr (Triplett), q (Quartett), H (Wasserstoffprotonen), J (Kopplungskonstante). Femer bedeuten: THF (Tetrahydrofuran), DMF (N,N-Dimethylformamid), MeOH (Methanol), EE (Ethylacetat), ml (Milliliter), RT (Raumtemperatur). Alle Lösemittel sind p.A.Qualität, wenn nicht anders vermerkt. Alle Reaktionen werden unter Schutzgas ausgeführt, es sei denn es handelt sich um wässrige Lösungen.

Nachfolgend wird die Darstellung einiger Vorstufen, Zwischenprodukte und Produkte exemplarisch beschrieben.

### Ausgangsverbindungen

### A1

Die Synthese von 6-Formyl-2-methyl-2H-1,4-benzoxazin-3-on wird in DE-198 26 232.9 beschrieben, ebenso die von
6-Formyl-2-ethyl-2H-1,4-benzoxazin-3-on und 6-Formyl-2-propyl-2H-1,4-benzoxazin-3-on.

### 6-((3-Aminomethyl)-benzylaminomethyl)-2-methyl-2H-1,4-benzoxazin-3-on und 6-(meta-(N-[3-Keto-2-methyl-2H-1,4-benzoxazin-6-yl]-methylaminomethyl)-benzylaminomethyl)-2-methyl-1,4-benzoxazin-3-on

In einer Mischung von 4 ml Methanol und 2 ml THF werden 382 mg 6-Formyl-2-methyl-1,4-benzoxazin-3-on gelöst und mit 136 mg 3-(Aminomethyl)-benzylamin versetzt. Man rührt 30 Minuten bei RT und gibt dann 101 mg Kaliumborhydrid hinzu. Nach 12 Stunden bei RT wird auf Wasser gegossen, dreimal extrahiert mit Ethylacetat und die organische Phase mit Sole gewaschen. Man trocknet mit Magnesiumsulfat und engt ein. Man erhält 455 mg Rohprodukt, das mit einer Schutzgruppe versehen und dann in einzelne Verbindungen durch Chromatographie aufgetrennt wird.

Auf die gleiche Weise werden hergestellt:
6-((4-Aminomethyl)-benzylaminomethyl)-2-methyl-2H-1,4-benzoxazin-3-on und
6-(para-(N-[3-Keto-2-methyl-2H-1,4-benzoxazin-6-yl)-methylaminomethyl)-benzylaminomethyl)-2-methyl-1,4-benzoxazin-3-on
6-(3-Aminopropyl-aminomethyl)-2-methyl-2H-1,4-benzoxazin-3(4H)-on
6-(3-[N-Methyl-amino]-propyl-aminomethyl)-2-methyl-2H-1,4-benzoxazin-3(4H)-on
6-(3-{[N-3-Chlorbenzyl]-aminopropyl}-aminomethyl)-2-methyl-2H-1,4-benzoxazin-3(4H)-on
6-(4-{[N-3-Chlorbenzyl]-amino-n-butyl}-aminomethyl)-2-methyl-2H-1,4-benzoxazin-3(4H)-on
6-(4-{[N-2-Thienylmethyl]-amino-n-butyl}-aminomethyl)-2-methyl-2H-1,4-benzoxazin-3(4H)-on
6-(5-{[N-3-Chlorbenzyl]-amino-n-pentyl}-aminomethyl)-2-methyl-2H-1,4-benzoxazin-3(4H)-on
6-(6-{[N-3-Chlorbenzyl]-amino-n-hexyl}-aminomethyl)-2-methyl-2H-1,4-benzoxazin-3(4H)-on
6-(4-{[N-4-Fluorbenzyl]-amino-n-butyl}-aminomethyl)-2-methyl-2H-1,4-benzoxazin-3(4H)-on
6-(4-{[N-3-Trifluorbenzyl]-amino-n-butyl}-aminomethyl)-2-methyl-2H-1,4-benzoxazin-3(4H)-on
6-(4-{[N-ortho-Hydroxybenzyl]-amino-n-butyl}-aminomethyl)-2-methyl-2H-1,4-benzoxazin-3(4H)-on
6-(5-{[N-Isopropyl]-amino-n-pentyl}-aminomethyl)-2-methyl-2H-1,4-benzoxazin-3(4H)-on 6-(4-{[N-Isopropyl]-amino-n-butyl}-aminomethyl)-2-methyl-2H-1,4-benzoxazin-3(4H)-on 6-(3-{[N-Isopropyl]-amino-n-propyl}-aminomethyl)-2-methyl-2H-1,4-benzoxazin-3(4H)-on
6-(4-{[N-Cyclopropyl]-amino-n-butyl}-aminomethyl)-2-methyl-2H-1,4-benzoxazin-3(4H)-on
6-(4-{[N-Cyclopentyl]-amino-n-butyl}-aminomethyl)-2-methyl-2H-1,4-benzoxazin-3(4H)-on
6-(4-{[N-(Cyclohexyl)-methyl]-amino-n-butyl}-aminomethyl)-2-methyl-2H-1,4-benzoxazin-3(4H)-on
6-(4-{[N-(Cyclopropyl)-methyl]-amino-n-butyl}-aminomethyl)-2-methyl-2H-1,4-benzoxazin-3(4H)-on
6-(4-{[N-2,2,2-Trifluorethyl]-amino-n-butyl}-aminomethyl)-2-methyl-2H-1,4-benzoxazin-3(4H)-on
6-(4-{[N-4,4,4-Trifluorbutyl]-amino-n-butyl}-aminomethyl)-2-methyl-2H-1,4-benzoxazin-3(4H)-on

Aus 6-Keto-6,7,8,9-tetrahydro-2-methyl-2H-naphth[2,3-b]-1,4-oxazin-3(4H)-on entstehen:
6-{[4-Amino-n-butyl]-amino}-6,7,8,9-tetrahydro-2-methyl-2H-naphth[2,3-b]-1,4-oxazin-3(4H)-on
6-{[5-Amino-n-pentyl]-amino}-6,7,8,9-tetrahydro-2-methyl-2H-naphth[2,3-b]-1,4-oxazin-3(4H)-on
6-{3-Aminomethyl-benzylamino}-6,7,8,9-tetrahydro-2-methyl-2H-naphth[2,3-b]-1,4-oxazin-3(4H)-on
6-{[4-(N-Isopropylamino)-n-butyl]-amino}-6,7,8,9-tetrahydro-2-methyl-2H-naphth[2,3-b]-1,4-oxazin-3(4H)-on
6-{[5-(N-Isopropylamino)-n-pentyl]-amino}-6,7,8,9-tetrahydro-2-methyl-2H-naphth[2,3-b]-1,4-oxazin-3(4H)-on

Aus 6-Keto-6,7-trimethylen-2-methyl-2H-1,4-benzoxazin-3(4H)-on entstehen:
6-{[4-Amino-n-butyl]-amino}-6,7-trimethylen-2-methyl-2H-1,4-benzoxazin-3(4H)-on
6-{[5-Amino-n-pentyl]-amino}-6,7-trimethylen-2-methyl-2H-1,4-benzoxazin-3(4H)-on
6-{[4-(N-Isopropylamino)-n-butyl]-amino}-6,7-trimethylen-2-methyl-2H-1,4-benzoxazin-3(4H)-on
6-{[5-(N-Isopropylamino)-n-pentyl]-amino}-6,7-trimethylen-2-methyl-2H-1,4-benzoxazin-3(4H)-on
6-{3-Aminomethyl-benzyl-amino}-6,7-trimethylen-2-methyl-2H-1,4-benzoxazin-3(4H)-on

Aus 1,3-Cyclohexyl-bis-methylamin:
6-((3-Aminomethyl-cyclohexy-1-yl)-methylaminomethyl)-2-methyl-2H-1,4-benzoxazin-3-on und 6-(3-(N-[3-Keto-2-methyl-2H-1,4-benzoxazin-6-yl]-methylaminomethyl)-cyclohex-1-ylmethylaminomethyl)-2-methyl-1,4-benzoxazin-3-on

Aus Diaminen:
6-((omega-Aminobutylaminomethyl)-2-methyl-2H-1,4-benzoxazin-3-on
6-((omega-Aminopentylaminomethyl)-2-methyl-2H-1,4-benzoxazin-3-on
6-((omega-Aminohexylaminomethyl)-2-methyl-2H-1,4-benzoxazin-3-on

### A2

### 6-((3-[4-Nitrobenzyl]-aminomethyl)-benzylaminomethyl)-2-methyl-2H-1,4-benzoxazin-3(4H)-on

In einer Mischung von 10 ml Methanol und 5 ml THF werden 573 mg 6-Formyl-2-methyl-1,4-benzoxazin-3-on gelöst und mit 0,382 ml 3-(Aminomethyl)-benzylamin sowie 438 mg p-Nitrobenzaldehyd versetzt. Man rührt 1 Stunde bei RT und gibt dann 173 mg Kaliumborhydrid hinzu. Nach 4 Stunden bei RT wird auf Wasser gegossen, dreimal extrahiert mit Ethylacetat und die organische Phase mit Sole gewaschen. Man trocknet mit Magnesiumsulfat und engt ein. Man erhält 1,18 g Rohprodukt, das mit einer Schutzgruppe versehen wird.

Auf die gleiche Weise werden hergestellt:
6-((3-[2-Methylbenzyl]-aminomethyl)-benzylaminomethyl)-2-methyl-2H-1,4-benzoxazin-3-on
6-((3-[2,4-Dichlorbenzyl]-aminomethyl)-benzylaminomethyl)-2-methyl-2H-1,4-benzoxazin-3-on
6-((3-[3-Chlorbenzyl]-aminomethyl)-benzylaminomethyl)-2-methyl-2H-1,4-benzoxazin-3-on
6-((3-[3,4-Dichlorbenzyl]-aminomethyl)-benzylaminomethyl)-2-methyl-2H-1,4-benzoxazin-3-on
6-((3-Benzylaminomethyl)-benzylaminomethyl)-2-methyl-2H-1,4-benzoxazin-3-on

### B

6-((3-[*tert*.-Butyloxycarbonyl]aminomethyl)-benzyl-(*tert*.-butyloxycarbonyl)-aminomethyl)-2-methyl-2H-1,4-benzoxazin-3-on **1** und
6-(meta-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-2H-1,4-benzoxazin-3-on **2**

**1**
[1H]-NMR (CDCl₃): 7,27 m 1H, 6,5 bis 7,18 m 7H, 5 breit 1H, 4,62 q 1H, 4,2 bis 4,4 m breit 5H, 1,58 d 3H, 1,50 s 9H 1,48 s 9H.
MS (ei) 511 m/z M+.
**2**
[1H]-NMR (CDCl₃): 7,1 bis 7,3 m breit und 6,6 bis 6,9 m zusammen 10H, 4,63 q 2H, 4,3 bis 4,4 m breit 8H, 1,6 d 6H, 1,50 s 18H.
MS (ei) 630, 586, 574, 529 m/z Fragmente.

Die Produkte erhält man durch Umsetzung der Mischung von 440 mg 6-((3-Aminomethyl)-benzylaminomethyl)-2-methyl-1,4-benzoxazin-3-on und 6-(meta-(N-[3-Keto-2-methyl-2H-1,4-benzoxazin-6-yl]-methylaminomethyl)-benzylaminomethyl)-2-methyl-1,4-benzoxazin-3-on in 15 ml Dichlormethan unter Zugabe von 0,38 ml Triethylamin und 476 mg Di-*tertiär*-butyldicarbonat. Nach 12 Stunden bei RT wird verdünnt mit Dichlormethan, gewaschen mit Natriumhydrogencarbonat und danach mit Sole. Man trocknet die org. Phase und engt ein. Nach Säulenchromatographie mit Hexan / Ethylacetat resultieren 160 mg **1** sowie 257 mg **2**.

Auf die gleiche Weise werden hergestellt:
6-((4-(*tert*.-butyloxycarbonyl)-aminomethyl)-benzyl-(*tert*.-butyloxycarbonyl)-aminomethyl)-2-methyl-1,4-benzoxazin-3-on und
6-(para-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-1,4-benzoxazin-3-on
6-((3-(*tert*.-butyloxycarbonyl)-aminomethyl-cyclohex-1-yl)-methyl-(*tert*.butyloxycarbonyl)aminomethyl)-2-methyl-1,4-benzoxazin-3-on und
6-(3-(N-[3-Keto-2-methyl-2H-1,4-benzoxazin-6-yl]-methyl-(*tert*.-butyloxycarbonyl)-aminomethyl)-cyclohex-1-ylmethyl-(*tert*.-butyloxycarbonyl)-aminomethyl)-2-methyl-1,4-benzoxazin-3-on

6-((omega-(*tert*.-butyloxycarbonyl)-aminobutyl-(*tert*.-butyloxycarbonyl)-aminomethyl)-2-methyl-1,4-benzoxazin-3-on
6-((omega-(*tert*.-butyloxycarbonyl)-aminopentyl-(*tert*.-butyloxycarbonyl)-aminomethyl)-2-methyl-1,4-benzoxazin-3-on
6-((omega-(*tert*.-butyloxycarbonyl)-aminohexyl-(*tert*.-butyloxycarbonyl)-aminomethyl)-2-methyl-1,4-benzoxazin-3-on
6-((3-[4-Nitrobenzyl]-(*tert*.-butyloxycarbonyl)-aminomethyl)-benzyl(*tert*.-butyloxycarbonyl)-aminomethyl)-2-methyl-1,4-benzoxazin-3-on
6-((3-[2-Methylbenzyl]-(*tert*.-butyloxycarbonyl)-aminomethyl)-benzyl(*tert*.-butyloxycarbonyl)-aminomethyl)-2-methyl-1,4-benzoxazin-3-on
6-((3-[2,4-Dichlorbenzyl]-(*tert*.-butyloxycarbonyl)-aminomethyl)-benzyl(*tert*.-butyloxycarbonyl)-aminomethyl)-2-methyl-1,4-benzoxazin-3-on
6-((3-[3-Chlorbenzyl]-(*tert*.-butyloxycarbonyl)-aminomethyl)-benzyl(*tert*.-butyloxycarbonyl)-aminomethyl)-2-methyl-1,4-benzoxazin-3-on
6-((3-[3,4-Dichlorbenzyl]-(*tert*.-butyloxycarbonyl)-aminomethyl)-benzyl(*tert*.-butyloxycarbonyl)-aminomethyl)-2-methyl-1,4-benzoxazin-3-on
6-((3-Benzyl(*tert*.-butyloxycarbonyl)-aminomethyl)-benzyl(*tert*.-butyloxycarbonyl)-aminomethyl)-2-methyl-1,4-benzoxazin-3-on

6-(3-(tert.-Butyloxycarbonyl)-aminopropyl-(tert.-butyloxycarbonyl)-aminomethyl)-2-methyl-2H-1,4-benzoxazin-3(4H)-on
6-(3-[N-Methyl-(tert.-butyloxycarbonyl)-amino]-propyl-(tert.-butyloxycarbonyl)-aminomethyl)-2-methyl-2H-1,4-benzoxazin-3(4H)-on
6-(3-{[N-3-Chlorbenzyl]-(tert.-butyloxycarbonyl)-aminopropyl}-(tert.-butyloxycarbonyl)-aminomethyl)-2-methyl-2H-1,4-benzoxazin-3(4H)-on
6-(4-{[N-3-Chlorbenzyl]-(tert.-butyloxycarbonyl)-amino-n-butyl}-(tert.-butyloxycarbonyl)-aminomethyl)-2-methyl-2H-1,4-benzoxazin-3(4H)-on
6-(4-{[N-2-Thienylmethyl]-(tert.-butyloxycarbonyl)-amino-n-butyl}-(tert.butyloxycarbonyl)-aminomethyl)-2-methyl-2H-1,4-benzoxazin-3(4H)-on
6-(5-{[N-3-Chlorbenzyl]-(tert.-butyloxycarbonyl)-amino-n-pentyl}-(tert.-butyloxycarbonyl)-aminomethyl)-2-methyl-2H-1,4-benzoxazin-3(4H)-on
6-(6-{[N-3-Chlorbenzyl]-(tert.-butyloxycarbonyl)-amino-n-hexyl}-(tert.-butyloxycarbonyl)-aminomethyl)-2-methyl-2H-1,4-benzoxazin-3(4H)-on
6-(4-{[N-4-Fluorbenzyl]-(tert.-butyloxycarbonyl)-amino-n-butyl}-(tert.-butyloxycarbonyl)-aminomethyl)-2-methyl-2H-1,4-benzoxazin-3(4H)-on
6-(4-{[N-3-Trifluorbenzyl]-(tert.-butyloxycarbonyl)-amino-n-butyl}-(tert.butyloxycarbonyl)-aminomethyl)-2-methyl-2H-1,4-benzoxazin-3(4H)-on
6-(4-{[N-ortho-Hydroxybenzyl]-(tert.-butyloxycarbonyl)-amino-n-butyl}-(tert.butyloxycarbonyl)-aminomethyl)-2-methyl-2H-1,4-benzoxazin-3(4H)-on
6-(5-{[N-Isopropyl]-(tert.-butyloxycarbonyl)-amino-n-pentyl}-(tert.-butyloxycarbonyl)-aminomethyl)-2-methyl-2H-1,4-benzoxazin-3(4H)-on
6-(4-{[N-Isopropyl]-(tert.-butyloxycarbonyl)-amino-n-butyl}-(tert.-butyloxycarbonyl)-aminomethyl)-2-methyl-2H-1,4-benzoxazin-3(4H)-on
6-(3-{[N-Isopropyl]-(tert.-butyloxycarbonyl)-amino-n-propyl}-(tert.-butyloxycarbonyl)-aminomethyl)-2-methyl-2H-1,4-benzoxazin-3(4H)-on
6-(4-{[N-Cyclopropyl]-(tert.-butyloxycarbonyl)-amino-n-butyl}-(tert.-butyloxycarbonyl)-aminomethyl)-2-methyl-2H-1,4-benzoxazin-3(4H)-on
6-(4-{[N-Cyclopentyl]-(tert.-butyloxycarbonyl)-amino-n-butyl}-(tert.-butyloxycarbonyl)-aminomethyl)-2-methyl-2H-1,4-benzoxazin-3(4H)-on
6-(4-{[N-(Cyclohexyl)-methyl]-(tert.-butyloxycarbonyl)-amino-n-butyl}-(tert.butyloxycarbonyl)-aminomethyl)-2-methyl-2H-1,4-benzoxazin-3(4H)-on
6-(4-{[N-(Cyclopropyl)-methyl]-(tert.-butyloxycarbonyl)-amino-n-butyl}-(tert.butyloxycarbonyl)-aminomethyl)-2-methyl-2H-1,4-benzoxazin-3(4H)-on
6-(4-{[N-2,2,2-Trifluorethyl]-(tert.-butyloxycarbonyl)-amino-n-butyl}-(tert.butyloxycarbonyl)-aminomethyl)-2-methyl-2H-1,4-benzoxazin-3(4H)-on
6-(4-{[N-4,4,4-Trifluorbutyl]-(tert.-butyloxycarbonyl)-amino-n-butyl}-(tert.butyloxycarbonyl)-aminomethyl)-2-methyl-2H-1,4-benzoxazin-3(4H)-on
6-{[4-(tert.-Butyloxycarbonyl)-amino-n-butyl]-(tert.-butyloxycarbonyl)-amino}-6,7,8,9-tetrahydro-2-methyl-2H-naphth[2,3-b]-1,4-oxazin-3(4H)-on
6-{[5-(tert.-Butyloxycarbonyl)-amino-n-pentyl]-(tert.-butyloxycarbonyl)-amino}-6,7,8,9-tetrahydro-2-methyl-2H-naphth[2,3-b]-1,4-oxazin-3(4H)-on
6-{3-(tert.-Butyloxycarbonyl)-aminomethyl-benzyl(tert.-butyloxycarbonyl)-amino}-6,7,8,9-tetrahydro-2-methyl-2H-naphth[2,3-b]-1,4-oxazin-3(4H)-on
6-{[4-(N-Isopropyl(tert.-butyloxycarbonyl)-amino)-n-butyl]-(tert.-butyloxycarbonyl)-amino}-6,7,8,9-tetrahydro-2-methyl-2H-naphth[2,3-b]-1,4-oxazin-3(4H)-on
6-{[5-(N-Isopropyl(tert.-butyloxycarbonyl)-amino)-n-pentyl]-(tert.-butyloxycarbonyl)-amino}-6,7,8,9-tetrahydro-2-methyl-2H-naphth[2,3-b]-1,4-oxazin-3(4H)-on
6-{[4-(tert.-Butyloxycarbonyl)-amino-n-butyl]-(tert.-butyloxycarbonyl)-amino}-6,7-trimethylen-2-methyl-2H-1,4-benzoxazin-3(4H)-on
6-{[5-(tert.-Butyloxycarbonyl)-amino-n-pentyl]-(tert.-butyloxycarbonyl)-amino}-6,7-trimethylen-2-methyl-2H-1,4-benzoxazin-3(4H)-on
6-{[4-(N-Isopropyl(tert.-butyloxycarbonyl)-amino)-n-butyl]-(tert.-butyloxycarbonyl)-amino}-6,7-trimethylen-2-methyl-2H-1,4-benzoxazin-3(4H)-on
6-{[5-(N-Isopropyl(tert.-butyloxycarbonyl)-amino)-n-pentyl]-(tert.-butyloxycarbonyl)-amino}-6,7-trimethylen-2-methyl-2H-1,4-benzoxazin-3(4H)-on
6-{3-(tert.-Butyloxycarbonyl)-aminomethyl-benzyl-(tert.-butyloxycarbonyl)-amino}-6,7-trimethylen-2-methyl-2H-1,4-benzoxazin-3(4H)-on

### C

### 6-((3-[tert.-Butyloxycarbonyl]-aminomethyl)-benzyl-(tert.-butyloxycarbonyl)-aminomethyl)-2-methyl-2H-1,4-benzoxazin-3(4H)-thion

Zu 150 mg 6-((3-[*tert*.-Butyloxycarbonyl]-aminomethyl)-benzyl-(*tert*.-butyloxycarbonyl)-aminomethyl)-2-methyl-1,4-benzoxazin-3-on in 12 ml Dimethoxyethan gibt man bei RT 192 mg Lawessons Reagenz und rührt 3 Stunden nach. Nach Einengen und Säulenchromatographie mit Hexan / Ethylacetat 4:1 resultieren 140 mg Produkt. Die Ausbeute ist 90%.
MS (ei) 527(M+) 471, 454, 427, 415, 370, 338 m/z Fragmente.

Auf die gleiche Weise werden hergestellt:
6-(meta-(N-[3-Thio-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-2H-1,4-benzoxazin-3(4H)-thion [MS (Cl-NH₃) 719 (M+H) Ausbeute 45%] bei 3 Equivalenten Lawessons Reagenz zusammen mit
6-(meta-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-2H-1,4-benzoxazin-3(4H)-thion
   Ausbeute 14 %.
6-((4-(*tert*.-butyloxycarbonyl)-aminomethyl)-benzyl-(*tert*.-butyloxycarbonyl)-aminomethyl)-2-methyl-2H-1,4-benzoxazin-3(4H)-thion
6-(para-(N-[3-Thio-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-2H-1,4-benzoxazin-3(4H)-thion
   46 % Ausbeute zusammen mit
6-(para-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-2-methyl-2H-1,4-benzoxazin-3(4H)-thion
6-((3-(*tert*.-butyloxycarbonyl)-aminomethyl-cyclohex-1-yl)-methyl-(*tert*.butyloxycarbonyl)aminomethyl)-2-methyl-2H-1,4-benzoxazin-3(4H)-thion
6-(3-(N-[3-Thio-2-methyl-2H-1,4-benzoxazin-6-yl]-methyl-(*tert*.-butyloxycarbonyl)-aminomethyl)-cyclohex-1-ylmethyl-(*tert*.-butyloxycarbonyl)-aminomethyl)-2-methyl-2H-1,4-benzoxazin-3(4H)-thion

6-((omega-(*tert*.-butyloxycarbonyl)-aminobutyl-(*tert*.-butyloxyarbonyl)-aminomethyl)-2-methyl-2H-1,4-benzoxazin-3(4H)-thion
6-((omega-(*tert*.-butyloxycarbonyl)-aminopentyl-(*tert*.-butyloxycarbonyl)-aminomethyl)-2-methyl-1,4-benzoxazin-3(4H)-thion
6-((omega-(*tert*.-butyloxycarbonyl)-aminohexyl-(*tert*.-butyloxycarbonyl)-aminomethyl)-2-methyl-2H-1,4-benzoxazin-3(4H)-thion
6-((3-[4-Nitrobenzyl]-(*tert*.-butyloxycarbonyl)-aminomethyl)-benzyl(*tert*.butyloxycarbonyl)-aminomethyl)-2-methyl-2H-1,4-benzoxazin-3(4H)-thion
6-((3-[2-Methylbenzyl]-(*tert*.-butyloxycarbonyl)-aminomethyl)-benzyl(*tert*.butyloxycarbonyl)-aminomethyl)-2-methyl-2H-1,4-benzoxazin-3(4H)-thion
6-((3-[2,4-Dichlorbenzyl]-(*tert*.-butyloxycarbonyl)-aminomethyl)-benzyl(*tert*.butyloxycarbonyl)-aminomethyl)-2-methyl-2H-1,4-benzoxazin-3(4H)-thion
6-((3-[3-Chlorbenzyl]-(*tert*.-butyloxycarbonyl)-aminomethyl)-benzyl(*tert*.butyloxycarbonyl)-aminomethyl)-2-methyl-2H-1,4-benzoxazin-3(4H)-thion
6-((3-[3,4-Dichlorbenzyl]-(*tert*.-butyloxycarbonyl)-aminomethyl)-benzyl(*tert*.butyloxycarbonyl)-aminomethyl)-2-methyl-2H-1,4-benzoxazin-3(4H)-thion
6-((3-Benzyl(*tert*.-butyloxycarbonyl)-aminomethyl)-benzyl(*tert*.-butyloxycarbonyl)-aminomethyl)-2-methyl-2H-1,4-benzoxazin-3(4H)-thion

6-(3-(tert.-Butyloxycarbonyl)-aminopropyl-(tert.-butyloxycarbonyl)-aminomethyl)-2-methyl-2H-1,4-benzoxazin-3(4H)-thion
6-(3-[N-Methyl-(tert.-butyloxycarbonyl)-amino]-propyl-(tert.-butyloxycarbonyl)-aminomethyl)-2-methyl-2H-1,4-benzoxazin-3(4H)-thion
6-(3-{[N-3-Chlorbenzyl]-(tert.-butyloxycarbonyl)-aminopropyl}-(tert.-butyloxycarbonyl)-aminomethyl)-2-methyl-2H-1,4-benzoxazin-3(4H)-thion
6-(4-{[N-3-Chlorbenzyl]-(tert.-butyloxycarbonyl)-amino-n-butyl}-(tert.-butyloxycarbonyl)-aminomethyl)-2-methyl-2H-1,4-benzoxazin-3(4H)-thion
6-(4-{[N-2-Thienylmethyl]-(tert.-butyloxycarbonyl)-amino-n-butyl}-(tert.butyloxycarbonyl)-aminomethyl)-2-methyl-2H-1,4-benzoxazin-3(4H)-thion
6-(5-{[N-3-Chlorbenzyl]-(tert.-butyloxycarbonyl)-amino-n-pentyl}-(tert.-butyloxycarbonyl)-aminomethyl)-2-methyl-2H-1,4-benzoxazin-3(4H)-thion
6-(6-{(N-3-Chlorbenzyl)-(tert.-butyloxycarbonyl)-amino-n-hexyl}-(tert.-butyloxycarbonyl)-aminomethyl)-2-methyl-2H-1,4-benzoxazin-3(4H)-thion
6-(4-{[N-4-Fluorbenzyl]-(tert.-butyloxycarbonyl)-amino-n-butyl}-(tert.-butyloxycarbonyl)-aminomethyl)-2-methyl-2H-1,4-benzoxazin-3(4H)-thion
6-(4-{[N-3-Trifluorbenzyl]-(tert.-butyloxycarbonyl)-amino-n-butyl}-(tert.butyloxycarbonyl)-aminomethyl)-2-methyl-2H-1,4-benzoxazin-3(4H)-thion
6-(4-{[N-ortho-Hydroxybenzyl]-(tert.-butyloxycarbonyl)-amino-n-butyl}-(tert.butyloxycarbonyl)-aminomethyl)-2-methyl-2H-1,4-benzoxazin-3(4H)-thion
6-(5-{[N-Isopropyl]-(tert.-butyloxycarbonyl)-amino-n-pentyl}-(tert.-butyloxycarbonyl)-aminomethyl)-2-methyl-2H-1,4-benzoxazin-3(4H)-thion
6-(4-{[N-Isopropyl]-(tert.-butyloxycarbonyl)-amino-n-butyl}-(tert.-butyloxycarbonyl)-aminomethyl)-2-methyl-2H-1,4-benzoxazin-3(4H)-thion
6-(3-{[N-Isopropyl]-(tert.-butyloxycarbonyl)-amino-n-propyl}-(tert.-butyloxycarbonyl)-aminomethyl)-2-methyl-2H-1,4-benzoxazin-3(4H)-thion
6-(4-{[N-Cyclopropyl]-(tert.-butyloxycarbonyl)-amino-n-butyl}-(tert.-butyloxycarbonyl)-aminomethyl)-2-methyl-2H-1,4-benzoxazin-3(4H)-thion
6-(4-{[N-Cyclopentyl]-(tert.-butyloxycarbonyl)-amino-n-butyl}-(tert.-butyloxycarbonyl)-aminomethyl)-2-methyl-2H-1,4-benzoxazin-3(4H)-thion
6-(4-{[N-(Cyclohexyl)-methyl]-(tert.-butyloxycarbonyl)-amino-n-butyl}-(tert.butyloxycarbonyl)-aminomethyl)-2-methyl-2H-1,4-benzoxazin-3(4H)-thion
6-(4-{[N-(Cyclopropyl)-methyl]-(tert.-butyloxycarbonyl)-amino-n-butyl}-(tert.butyloxycarbonyl)-aminomethyl)-2-methyl-2H-1,4-benzoxazin-3(4H)-thion
6-(4-{[N-2,2,2-Trifluorethyl]-(tert.-butyloxycarbonyl)-amino-n-butyl}-(tert.butyloxycarbonyl)-aminomethyl)-2-methyl-2H-1,4-benzoxazin-3(4H)-thion
6-(4-{[N-4,4,4-Trifluorbutyl]-(tert.-butyloxycarbonyl)-amino-n-butyl}-(tert.butyloxycarbonyl)-aminomethyl)-2-methyl-2H-1,4-benzoxazin-3(4H)-thion
6-{[4-(tert.-Butyloxycarbonyl)-amino-n-butyl]-(tert.-butyloxycarbonyl)-amino}-6,7,8,9-tetrahydro-2-methyl-2H-naphth[2,3-b]-1,4-oxazin-3(4H)-thion
6-{[5-(tert.-Butyloxycarbonyl)-amino-n-pentyl]-(tert.-butyloxycarbonyl)-amino}-6,7,8,9-tetrahydro-2-methyl-2H-naphth[2,3-b]-1,4-oxazin-3(4H)-thion
6-{3-(tert.-Butyloxycarbonyl)-aminomethyl-benzyl(tert.-butyloxycarbonyl)-amino}-6,7,8,9-tetrahydro-2-methyl-2H-naphth[2,3-b]-1,4-oxazin-3(4H)-thion
6-{[4-(N-Isopropyl(tert.-butyloxycarbonyl)-amino)-n-butyl]-(tert.-butyloxycarbonyl)-amino}-6,7,8,9-tetrahydro-2-methyl-2H-naphth[2,3-b]-1,4-oxazin-3(4H)-thion
6-{[5-(N-Isopropyl(tert.-butyloxycarbonyl)-amino)-n-pentyl]-(tert.-butyloxycarbonyl)-amino}-6,7,8,9-tetrahydro-2-methyl-2H-naphth[2,3-b]-1,4-oxazin-3(4H)-thion
6-{[4-(tert.-Butyloxycarbonyl)-amino-n-butyl]-(tert.-butyloxycarbonyl)-amino}-6,7-trimethylen-2-methyl-2H-1,4-benzoxazin-3(4H)-thion
6-{[5-(tert.-Butyloxycarbonyl)-amino-n-pentyl]-(tert.-butyloxycarbonyl)-amino}-6,7-trimethylen-2-methyl-2H-1,4-benzoxazin-3(4H)-thion
6-{[4-(N-Isopropyl(tert.-butyloxycarbonyl)-amino)-n-butyl}-(tert.-butyloxycarbony)-amino}-6,7-trimethylen-2-methyl-2H-1,4-benzoxazin-3(4H)-thion
6-{[5-(N-Isopropyl(tert.-butyloxycarbonyl)-amino)-n-pentyl]-(tert.-butyloxycarbonyl)-amino}-6,7-trimethylen-2-methyl-2H-1,4-benzoxazin-3(4H)-thion
6-{3-(tert.-Butyloxycarbonyl)-aminomethyl-benzyl-(tert.-butyloxycarbonyl)-amino}-6,7-trimethylen-2-methyl-2H-1,4-benzoxazin-3(4H)-thion

### Beispiel 1

### 6-((3-[tert.-Butyloxycarbonyl]-aminomethyl)-benzyl-(tert.-butyloxycarbonyl)-aminomethyl)-3-amino-2-methyl-2H-1,4-benzoxazin

Man rührt 140 mg 6-((3-[*tert*.-Butyloxycarbonyl]-aminomethyl)-benzyl-(*tert*.-butyloxycarbonyl)-aminomethyl)-2-methyl-1,4-benzoxazin-3-thion in 50 ml ges. Ammoniaklösung in Methanol (kommerziell erhältlich). Nach 1 Tag bei Raumtemperatur erhält man das Rohprodukt nach Einengen. Säulenchromatografie mit Essigester reinigt das Produkt. Es resultieren 75 % Ausbeute.
[1H]-NMR (DMSO): 7,30 dd 2H, 7,14 dd 2H, 7,08 d 1H, 6,6 bis 6,75 m 4H inclusive Amidin NH, 4,62 q 1H, 4,35 s breit 2H, 4,22 s breit 2H, 4,15 s breit 2H, 1,42 s 9H, 1,40 s 9H, 1,28 d 3H.
MS (ei): 510 m/z (M+).

Auf die gleiche Weise werden hergestellt:
6-(meta-(N-[3-Amino-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-3-amino-2-methyl-2H-1,4-benzoxazin
   95 % Ausbeute.
   [1H]-NMR (DMSO): 7,30 dd 1H, 7,10 m 3H, 6,6 bis 6,75 m 10H inclusive Amidin NH, 4,64 q 2H, 4,30 s breit 4H, 4,21 s breit 4H, 1,42 s 18H, 1,29 d 6H.
   MS (Cl-NH₃) 685 m/z (M+1)
6-(meta-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-3-amino-2-methyl-2H-1,4-benzoxazin
   90% Ausbeute
   MS (Cl-NH₃) 686 m/z (M+1)
6-((4-(*tert*.-butyloxycarbonyl)-aminomethyl)-benzyl-(*tert*.-butyloxycarbonyl)-aminomethyl)-3-amino-2-methyl-2H-1,4-benzoxazin
6-(para-(N-[3-Amino-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-3-amino-2-methyl-2H-1,4-benzoxazin
   92%, [1H]-NMR (DMSO): 7,17 s 2H, 6,6 bis 6,75 m 8H, 4,64 q 2H, 4,30 s breit 4H, 4,21 s breit 4H, 1,41 s 18H, 1,28 d 6H.
   MS (Cl-Thioglycerin) 685 m/z (M+1)
6-(para-(N-[3-Keto-2-methyl-2H-1,4-benzoxazin-6-yl]-methyl-(*tert*.-butyloxycarbonyl)-aminomethyl)-benzyl-(*tert*.-butyloxycarbonyl)-aminomethyl)-3-amino-2-methyl-2H-1,4-benzoxazin
6-((3-(*tert*.-butyloxycarbonyl)-aminomethyl-cyclohex-1-yl)-methyl-(*tert*.butyloxycarbonyl)aminomethyl)-3-amino-2-methyl-2H-1,4-benzoxazin
   MS (Cl-Thioglycerin) 517 m/z (M+1 )
6-(3-(N-[3-Amino-2-methyl-2H-1,4-benzoxazin-6-yl]-methyl-(*tert*.-butyloxycarbonyl)-aminomethyl)-cyclohex-1-ylmethyl-(*tert*.-butyloxycarbonyl)-aminomethyl)-3-amino-2-methyl-2H-1,4-benzoxazin

6-((omega-(*tert*.-butyloxycarbonyl)-aminobutyl-(*tert*.-butyloxycarbonyl)-aminomethyl)-3-amino-2-methyl-2H-1,4-benzoxazin
6-((omega-(*tert*.-butyloxycarbonyl)-aminopentyl-(*tert*.-butyloxycarbonyl)-aminomethyl)-3-amino-2-methyl-2H-1,4-benzoxazin
6-((omega-(*tert*.-butyloxycarbonyl)-aminohexyl-(*tert*.-butyloxycarbonyl)-aminomethyl)-3-amino-2-methyl-2H-1,4-benzoxazin
6-((3-[4-Nitrobenzyl]-(*tert*.-butyloxycarbonyl)-aminomethyl)-benzyl(*tert*.butyloxycarbonyl)-aminomethyl)-3-amino-2-methyl-2H-1,4-benzoxazin
6-((3-[2-Methylbenzyl]-(*tert*.-butyloxycarbonyl)-aminomethyl)-benzyl(*tert*.butyloxycarbonyl)-aminomethyl)-3-amino-2-methyl-2H-1,4-benzoxazin
6-((3-[2,4-Dichlorbenzyl]-(*tert*.-butyloxycarbonyl)-aminomethyl)-benzyl(*tert*.butyloxycarbonyl)-aminomethyl)-3-amino-2-methyl-2H-1,4-benzoxazin
6-((3-[3-Chlorbenzyl]-(*tert*.-butyloxycarbonyl)-aminomethyl)-benzyl(*tert*.butyloxycarbonyl)-aminomethyl)-3-amino-2-methyl-2H-1,4-benzoxazin
6-((3-[3,4-Dichlorbenzyl]-(*tert*.-butyloxycarbonyl)-aminomethyl)-benzyl(*tert*.butyloxycarbonyl)-aminomethyl)-3-amino-2-methyl-2H-1,4-benzoxazin
6-((3-Benzyl(*tert*.-butyloxycarbonyl)-aminomethyl)-benzyl(*tert*.-butyloxycarbonyl)-aminomethyl)-3-amino-2-methyl-2H-1,4-benzoxazin

6-(3-(tert.-Butyloxycarbonyl)-aminopropyl-(tert.-butyloxycarbonyl)-aminomethyl)-3-amino-2-methyl-2H-1,4-benzoxazin
6-(3-[N-Methyl-(tert.-butyloxycarbonyl)-amino]-propyl-(tert.-butyloxycarbonyl)-aminomethyl)-3-amino-2-methyl-2H-1,4-benzoxazin
6-(3-{[N-3-Chlorbenzyl]-(tert.-butyloxycarbonyl)-aminopropyl}-(tert.-butyloxycarbonyl)-aminomethyl)-3-amino-2-methyl-2H-1,4-benzoxazin
6-(4-{[N-3-Chlorbenzyl]-(tert.-butyloxycarbonyl)-amino-n-butyl}-(tert.-butyloxycarbonyl)-aminomethyl)-3-amino-2-methyl-2H-1,4-benzoxazin
6-(4-{[N-2-Thienylmethyl]-(tert.-butyloxycarbonyl)-amino-n-butyl}-(tert.butyloxycarbonyl)-aminomethyl)-3-amino-2-methyl-2H-1,4-benzoxazin
6-(5-{[N-3-Chlorbenzyl]-(tert.-butyloxycarbonyl)-amino-n-pentyl}-(tert.-butyloxycarbonyl)-aminomethyl)-3-amino-2-methyl-2H-1,4-benzoxazin
6-(6-{[N-3-Chlorbenzyl]-(tert.-butyloxycarbonyl)-amino-n-hexyl}-(tert.-butyloxycarbonyl)-aminomethyl)-3-amino-2-methyl-2H-1,4-benzoxazin
6-(4-{[N-4-Fluorbenzyl]-(tert.-butyloxycarbonyl)-amino-n-butyl}-(tert.-butyloxycarbonyl)-aminomethyl)-3-amino-2-methyl-2H-1,4-benzoxazin
6-(4-{[N-3-Trifluorbenzyl]-(tert.-butyloxycarbonyl)-amino-n-butyl}-(tert.butyloxycarbonyl)-aminomethyl)-3-amino-2-methyl-2H-1,4-benzoxazin
6-(4-{[N-ortho-Hydroxybenzyl]-(tert.-butyloxycarbonyl)-amino-n-butyl}-(tert.butyloxycarbonyl)-aminomethyl)-3-amino-2-methyl-2H-1,4-benzoxazin
6-(5-{[N-Isopropyl]-(tert.-butyloxycarbonyl)-amino-n-pentyl}-(tert.-butyloxycarbonyl)-aminomethyl)-3-amino-2-methyl-2H-1,4-benzoxazin
6-(4-{[N-Isopropyl]-(tert.-butyloxycarbonyl)-amino-n-butyl}-(tert.-butyloxycarbonyl)-aminomethyl)-3-amino-2-methyl-2H-1,4-benzoxazin
6-(3-{[N-Isopropyl]-(tert.-butyloxycarbonyl)-amino-n-propyl}-(tert.-butyloxycarbonyl)-aminomethyl)-3-amino-2-methyl-2H-1,4-benzoxazin
6-(4-{[N-Cyclopropyl]-(tert.-butyloxycarbonyl)-amino-n-butyl}-(tert.-butyloxycarbonyl)-aminomethyl)-3-amino-2-methyl-2H-1,4-benzoxazin
6-(4-{[N-Cyclopentyl]-(tert.-butyloxycarbonyl)-amino-n-butyl}-(tert.-butyloxycarbonyl)-aminomethyl)-3-amino-2-methyl-2H-1,4-benzoxazin
6-(4-{[N-(Cyclohexyl)-methyl]-(tert.-butyloxycarbonyl)-amino-n-butyl}-(tert.butyloxycarbonyl)-aminomethyl)-3-amino-2-methyl-2H-1,4-benzoxazin
6-(4-{[N-(Cyclopropyl)-methyl]-(tert.-butyloxycarbonyl)-amino-n-butyl}-(tert.butyloxycarbonyl)-aminomethyl)-3-amino-2-methyl-2H-1,4-benzoxazin
6-(4-{[N-2,2,2-Trifluorethyl]-(tert.-butyloxycarbonyl)-amino-n-butyl}-(tert.butyloxycarbonyl)-aminomethyl)-3-amino-2-methyl-2H-1,4-benzoxazin
6-(4-{[N-4,4,4-Trifluorbutyl]-(tert.-butyloxycarbonyl)-amino-n-butyl}-(tert.butyloxycarbonyl)-aminomethyl)-3-amino-2-methyl-2H-1,4-benzoxazin
6-{[4-(tert.-Butyloxycarbonyl)-amino-n-butyl]-(tert.-butyloxycarbonyl)-amino}-6,7,8,9-tetrahydro-3-amino-2-methyl-2H-naphth[2,3-b]-1,4-oxazin
6-{[5-(tert.-Butyloxycarbonyl)-amino-n-pentyl]-(tert.-butyloxycarbonyl)-amino}-6,7,8,9-tetrahydro-3-amino-2-methyl-2H-naphth[2,3-b]-1,4-oxazin
6-{3-(tert.-Butyloxycarbonyl)-aminomethyl-benzyl(tert.-butyloxycarbonyl)-amino}-6,7,8,9-tetrahydro-3-amino-2-methyl-2H-naphth[2,3-b]-1,4-oxazin
6-{[4-(N-Isopropyl(tert.-butyloxycarbonyl)-amino)-n-butyl]-(tert.-butyloxycarbonyl)-amino}-6,7,8,9-tetrahydro-3-amino-2-methyl-2H-naphth[2,3-b]-1,,4-oxazin
6-{[5-(N-Isopropyl(tert.-butyloxycarbonyl)-amino)-n-pentyl]-(tert.-butyloxycarbonylamino}-6,7,8,9-tetrahydro-3-amino-2-methyl-2H-naphth[2,3]-bi-1,4-oxazin
6-{[4-(tert.-Butyloxycarbonyl)-amino-n-butyl]-(tert.-butyloxycarbonyl)-amino}-6,7-trimethylen-3-amino-2-methyl-2H-1,4-benzoxazin
6-{[5-(tert.-Butyloxycarbonyl)-amino-n-pentyl]-(tert.-butyloxycarbonyl)-amino}-6,7-trimethylen-3-amino-2-methyl-2H-1,4-benzoxazin
6-{[4-(N-Isopropyl(tert.-butyloxycarbonyl)-amino)-n-butyl]-(tert.-butyloxycarbonyl)-amino}-6,7-trimethylen-3-amino-2-methyl-2H-1,4-benzoxazin
6-{[5-(N-Isopropyl(tert.-butyloxycarbonyl)-amino)-n-pentyl]-(tert.-butyloxycarbonyl)-amino}-6,7-trimethylen-3-amino-2-methyl-2H-1,4-benzoxazin
6-{3-(tert.-Butyloxycarbonyl)-aminomethyl-benzyl-(tert.-butyloxycarbonyl)-amino}-6,7-trimethylen-3-amino-2-methyl-2H-1,4-benzoxazin

### Beispiel 2

### 6-((3-aminomethyl)-benzyl-aminomethyl)-3-amino-2-methyl-2H-1,4-benzoxazin Trihydrochlorid

95 mg von 6-((3-[*tert*.-Butyloxycarbonyl]-aminomethyl)-benzyl-(*tert*.-butyloxycarbonyl)-aminomethyl)-3-amino-2-methyl-1,4-benzoxazin werden in 3 ml Dioxan mit 2 ml 4 n Salzsäure (Lösung in Dioxan) gerührt. Nach 12 Stunden wird mit etwas Ethylacetat verdünnt, die Kristalle abgesaugt, mit wenig Ethylacetat gewaschen und im Vakuum getrocknet. Man erhält 66 mg Produkt (92 % Ausbeute).
[1H]-NMR (DMSO): 9,9 breit, 9,5 breit , 8,5 breit s, 7,37 bis 7,70 m 6 H, 7,11 d 1H, 5,36 q 1H, 4,15 breit 2H, 4,14 breit 2H, 4,04 breit 2H, 1,50 d 3H.

Auf die gleiche Weise werden hergestellt:
6-(meta-(N-[3-Keto-2-methyl-2H-1,4-benzoxazin-6-yl]-methyl-aminomethyl)-benzylaminomethyl)-3-amino-2-methyl-2H-1,4-benzoxazin Trihydrochlorid
   Ausbeute 99 %
   [1H]-NMR (DMSO): 9,9 breit, 9,7 breit , 7,0 bis 7,75 m 10H, 5,33 q 1H, 4,70 q 1H, 4,15 breit 4H, 4,1 m 4H 1,50 d 3H, 1,44 d 3H.
6-(meta-(N-[3-Amino-2-methyl-2H-1,4-benzoxazin-6-yl]-methyl-aminomethyl)-benzylaminomethyl)-3-amino-2-methyl-2H-1,4-benzoxazin Trihydrochlorid (HCl Gehalt nicht bestimmt)
   Ausbeute 87 %.
   [1H]-NMR (DMSO): 9,9 breit, 9,5 breit , 7,38 dd 2H, 7,5 m 3H, 7,65 dd 2H, 7,75 s 1H , 7,11 d 2H, 5,33 q 2H, 4,15 breit 8H, 1,50 d 6H.
6-((4-aminomethyl)-benzyl-aminomethyl)-3-amino-2-methyl-2H-1,4-benzoxazin Trihydrochlorid
6-(para-(N-[3-Amino-2-methyl-2H-1,4-benzoxazin-6-yl]-methyl-aminomethyl)-benzylaminomethyl)-3-amino-2-methyl-2H-1,4-benzoxazin Trihydrochlorid (HCl Gehalt nicht bestimmt)
   [1H]-NMR (DMSO): 9,9 breit, 9,5 breit, 7,64 s 4H, 7,48 dd 2H, 7,35 dd 2H , 7,12 d 2H, 5,33 q 2H, 4,19 breit 4H, 4,11 breit 4H, 1,50 d 6H.
6-(para-(N-[3-Keto-2-methyl-2H-1,4-benzoxazin-6-yl]-methyl-aminomethyl)-benzylaminomethyl)-3-amino-2-methyl-2H-1,4-benzoxazin Trihydrochlorid 6-((3-aminomethyl-cyclohex-1-yl)-methyl-aminomethyl)-3-amino-2-methyl-2H-1,4-benzoxazin Trihydrochlorid
   [1H]-NMR (DMSO): 9,4 breit, 8,1 breit s, 7,5 d 1H, 7,43 d 1H, 7,12 d 1H, 5,34 q 1H, 4,12 breit 2H, 1,2 bis 2,9 m 14H, 1,51 d 3H.
6-(3-(N-[3-Amino-2-methyl-2H-1,4-benzoxazin-6-yl]-methyl-aminomethyl)-cyclohex-1-ylmethyl-aminomethyl)-3-amino-2-methyl-2H-1,4-benzoxazin Trihydrochlorid
   (HCl Gehalt nicht bestimmt)
6-((omega-aminobutyl-aminomethyl)-3-amino-2-methyl-2H-1,4-benzoxazin Trihydrochlorid
6-((omega-aminopentyl-aminomethyl)-3-amino-2-methyl-2H-1,4-benzoxazin Trihydrochlorid
   Ausbeute 84%
   [1H]-NMR (DMSO): 13,2 breit, 10,1 breit, 9,0 breit , 7,48 d 1H, 7,37 d 1H, 7,13 d 1H, 5,34 q 1H, 4,10 breit 2H, 2,7 bis 2,9 m 4H, 0,85 bis 1,78 m 6H, 1,50 d 3H.
6-((omega-aminohexyl-aminomethyl)-3-amino-2-methyl-2H-1,4-benzoxazin Trihydrochlorid
6-((3-[4-Nitrobenzyl]-aminomethyl)-benzylaminomethyl)-3-amino-2-methyl-2H-1,4-benzoxazin Trihydrochlorid
   [1H]-NMR (DMSO): 9,9 breit, 8,28 d 2H, 7,92 d 2H, 7,77 s 1H, 7,65 d 2H, 7,5 m 2H, 7,39 m 1H, 7,11 d 1H, 5,34 q 1H, 4,35 breit s 2H, 4,21 s 2H, 4,16 s 4H, 1,51 d 3H.
6-((3-[2-Methylbenzyl]-aminomethyl)-benzylaminomethyl)-3-amino-2-methyl-2H-1,4-benzoxazin Trihydrochlorid
6-((3-[2,4-Dichlorbenzyl]-aminomethyl)-benzylaminomethyl)-3-amino-2-methyl-2H-1,4-benzoxazin Trihydrochlorid
6-((3-[3-Chlorbenzyl]-aminomethyl)-benzylaminomethyl)-3-amino-2-methyl-2H-1,4-benzoxazin Trihydrochlorid
6-((3-[3,4-Dichlorbenzyl]-aminomethyl)-benzylaminomethyl)-3-amino-2-methyl-2H-1,4-benzoxazin Trihydrochlorid
6-((3-Benzylaminomethyl)-benzylaminomethyl)-3-amino-2-methyl-2H-1,4-benzoxazin Trihydrochlorid

6-(3-Aminopropyl-aminomethyl)-3-amino-2-methyl-2H-1,4-benzoxazin Trihydrochlorid 6-(3-[N-Methylamino]-propyl-aminomethyl)-3-amino-2-methyl-2H-1,4-benzoxazin Trihydrochlorid
   [1H]-NMR (DMSO): 9 -10 m breit NH, 7,49 d 1H, 7,40 dd 1H, 7,12 d 1H, 5,39 q 1H, 4,11 d 2H, 2,5 s 3H, 3,05 m 4H, 2,12 m 2H, 1,51 d 3H.
6-(3-{[N-3-Chlorbenzyl]-aminopropyl}-aminomethyl)-3-amino-2-methyl-2H-1,4-benzoxazin Trihydrochlorid
   [1H]-NMR (DMSO): 9,6 m breit, 7,74 s 1H, 7,57 dd 1H, 7,49 d 3H, 7,39 d 1H, 7,12 d 1H, 5,35 q 1H, 4,15 s 2H, 4,10 s breit 2H, 3,05 m 4H, 2,15 m 2H, 1,49 d 3H.
6-(4-{[N-3-Chlorbenzyl]-amino-n-butyl}-aminomethyl)-3-amino-2-methyl-2H-1,4-benzoxazin Trihydrochlorid
   [1H]-NMR (DMSO): 9,5 m breit, 7,75 s 1H, 7,57 dd 1H, 7,49 m 3H, 7,39dd 1H, 7,12 d 1H, 5,37 q 1H, 4,10 d breit 4H, 2,9 m 4H, 1,74 m 4H, 1,49 d 3H.
6-(4-{[N-2-Thienylmethyl]-amino-n-butyl}-aminomethyl)-3-amino-2-methyl-2H-1,4-benzoxazin Trihydrochlorid
   [1H]-NMR (DMSO): 9,5 m breit, 7,63 d 1H, 7,47 s 1H, 7,38 dd 2H, 7,1 m 2H, 5,34 q 1H, 4,35 s breit 2H, 4,09 s breit 2H, 2,9 m 4H, 1,75 m 4H, 1,50 d 3H.
6-(5-{[N-3-Chlorbenzyl]-amino-n-pentyl}-aminomethyl)-3-amino-2-methyl-2H-1,4-benzoxazin Trihydrochlorid
   [1H]-NMR (DMSO): 9,5 m breit, 7,74 s 1H, 7,56 dd 1H, 7,47 m 3H, 7,38dd 1H, 7,11 d 1H, 5,35 q 1H, 4,15 d 2H, 4,09 d 2H, 2,9 m 4H, 1,72 m 4H, 1,51 d 3H, 1,4 m 2H.
6-(6-{[N-3-Chlorbenzyl]-amino-n-hexyl}-aminomethyl)-3-amino-2-methyl-2H-1,4-benzoxazin Trihydrochlorid
6-(4-{[N-4-Fluorbenzyl]-amino-n-butyl}-aminomethyl)-3-amino-2-methyl-2H-1,4-benzoxazin Trihydrochlorid
   [1H]-NMR (DMSO): 9,5 m breit, 7,67 m 2H, 7,48 s 1H, 7,39 dd 1H, 7,29 dd 2H, 7,12 d 1H, 5,37 q 1H, 4,1 m 4H, 2,9 m 4H, 1,75 m 4H, 1,50 d 3H.
6-(4-{[N-3-Trifluorbenzyl]-amino-n-butyl}-aminomethyl)-3-amino-2-methyl-2H-1,4-benzoxazin Trihydrochlorid
   [1H]-NMR (DMSO): 9,6 m breit, ( 8,05 s, 7,93 d, 7,8 d, 7,7 dd, 7,47 s, 7,39d, 7,13 d jeweils 1H), 5,36 q 1H, 4,25 breit 2H, 4,10 breit 2H, 2,9 m 4H, 1,78 m 4H, 1,50 d 3H.
6-(4-{[N-ortho-Hydroxybenzyl]-amino-n-butyl}-aminomethyl)-3-amino-2-methyl-2H-1,4-benzoxazin Trihydrochlorid
6-(5-{[N-Isopropyl]-amino-n-pentyl}-aminomethyl)-3-amino-2-methyl-2H-1,4-benzoxazin Trihydrochlorid
   [1H]-NMR (DMSO): 9,5 m breit, 9,9 m breit NH, 7,49 d 1H, 7,39dd 1H, 7,13 d 1H, 5,37 q 1H, 4,10 s 2H, 3,2 hept 1H, 2,85 m 4H, 1,7 m 4H, 1,4 m 2H, 1,51 d 3H, 1,26 d 6H.
6-(4-{[N-Isopropyl]-amino-n-butyl}-aminomethyl)-3-amino-2-methyl-2H-1,4-benzoxazin Trihydrochlorid
   [1H]-NMR (DMSO): 9,5 m breit, 9 m breit NH, 7,48 d 1H, 7,39 dd 1H, 7,12 d 1H, 5,35 q 1H, 4,11 s 2H, 3,25 hept 1H, 2,9 m 4H, 1,75 m 4H, 1,5 d 3H, 1,27 d 6H.
6-(3-{[N-Isopropyl]-amino-n-propyl}-aminomethyl)-3-amino-2-methyl-2H-1,4-benzoxazin Trihydrochlorid
   [1H]-NMR (DMSO): 9,5 m breit, 9,1 m breit NH, 7,49 d 1H, 7,4 dd 1H, 7,12 d 1H, 5,35 q 1H, 4,11 s 2H, 3,3 hept 1H, 3,0 m 4H, 2,1 m 2H, 1,5 d 3H, 1,27 d 6H.
6-(4-{[N-Cyclopropyl]-amino-n-butyl}-aminomethyl)-3-amino-2-methyl-2H-1,4-benzoxazin Trihydrochlorid
6-(4-{[N-Cyclopentyl]-amino-n-butyl}-aminomethyl)-3-amino-2-methyl-2H-1,4-benzoxazin Trihydrochlorid
   [1H]-NMR (DMSO): 9,5 m breit, 9,1 m breit NH, 7,48 d 1H, 7,39 dd 1H, 7,12 d 1H, 5,37 q 1H, 4,09 s 2H, 2,9 m 4H, 1,95 m 2H, 1,7 m 8H, 1,50 d 3H, 1,52 m 1H.
6-(4-{[N-(Cyclohexyl)-methyl]-amino-n-butyl}-aminomethyl)-3-amino-2-methyl-2H-1,4-benzoxazin Trihydrochlorid
   [1H]-NMR (DMSO): 9 -10 m breit NH, 7,49 d 1H, 7,39 dd 1H, 7,12 d 1H, 5,37 q 1H, 4,10 d 2H, 2,91 m 4H, 2,74 m 2H, 1,85 bis 1,6 m 10H, 1,50 d 3H, 1,3 bis 0,85 m 5H.
6-(4-{[N-(Cyclopropyl)-methyl]-amino-n-butyl}-aminomethyl)-3-amino-2-methyl-2H-1,4-benzoxazin Trihydrochlorid

6-(4-{[N-2,2,2-Trifluorethyl]-amino-n-butyl}-aminomethyl)-3-amino-2-methyl-2H-1,4-benzoxazin Trihydrochlorid
   [1H]-NMR (DMSO): 9,5 m breit, 7,49 d 1H, 7,39 dd 1H, 7,11 d 1H, 5,37 q 1H, 4,1 s 2H, 3,99 m 2H (*CH*₂CF₃), 3,02 m 2H, 2,92 m 2H, 1,75 m 4H, 1,50 d 3H.
6-(4-{[N-4,4,4-Trifluorbutyl]-amino-n-butyl}-aminomethyl)-3-amino-2-methyl-2H-1,4-benzoxazin Trihydrochlorid
6-{[4-Amino-n-butyl]-amino}-6,7,8,9-tetrahydro-3-amino-2-methyl-2H-naphth[2,3-b]-1,4-oxazin Trihydrochlorid
6-{[5-Amino-n-pentyl]-amino}-6,7,8,9-tetrahydro-3-amino-2-methyl-2H-naphth[2,3-b]-1,4-oxazin Trihydrochlorid

6-{[3-Aminomethyl]-benzylamino}-6,7,8,9-tetrahydro-3-amino-2-methyl-2H-naphth[2,3-b]-1,4-oxazin Trihydrochlorid
   [1H]-NMR (DMSO): 9,5 bis 8,5 m breit, 7,9 s 1H, 7,8 d 1H, 7,64 d 1H, 7,53 m 2H, 7,0 s 1H, 5,41 q 1H, 4,5 m 1H, 4,3 s 2H, 4,1 s 2H, 2,9 m 2H, 2,3 m 1H, 2,1 m 2H, 1,8 m 1H, 1,55 d 3H.
6-{[4-(N-Isopropylamino)-n-butyl]-amino}-6,7,8,9-tetrahydro-3-amino-2-methyl-2Hnaphth[2,3-b]-1,4-oxazin Trihydrochlorid
6-{[5-(N-Isopropylamino)-n-pentyl]-amino}-6,7,8,9-tetrahydro-3-amino-2-methyl-2Hnaphth[2,3-b]-1,4-oxazin Trihydrochlorid
   [1H]-NMR(MeOH): 7,34 s 1H, 6,85 s 1H, 5,13 q 1H, 4,4 m 1H, 3,29 m 1H, 3,05 dtr 2H, 2,92 m 2H, 2,8 m 2H, 2,15 m 1H, 2,0 m 1H, 1,8 m 2H, 1,7 m 2H, 1,6 m 2H, 1,47 d 3H, 1,24 d 6H.
6-{[4-Amino-n-butyl]-amino}-6,7-trimethylen-3-amino-2-methyl-2H-1,4-benzoxazin Trihydrochlorid
6-{[5-Amino-n-pentyl]-amino}-6,7-trimethylen-3-amino-2-methyl-2H-1,4-benzoxazin Trihydrochlorid
6-{[4-(N-Isopropylamino)-n-butyl]-amino}-6,7-trimethylen-3-amino-2-methyl-2H-1,4-benzoxazin Trihydrochlorid
   [1H]-NMR(MeOH): 7,51 s 1H, 7,0 s 1H, 5,13 q 1H, 4,7 m 1H, 3,3 m 1H, 3,1 m 2H, 3,0 m 2H,2,9 m 2H, 2,5 m 1H, 2,2 m 1H, 1,8 m 2H, 1,7 m 2H, 1,49 d 3H, 1,27 d 6H.
6-{[5-(N-Isopropylamino)-n-pentyl]-amino}-6,7-trimethylen-3-amino-2-methyl-2H-1,4-benzoxazin Trihydrochlorid
6-{[3-Aminomethyl]-benzylamino}-6,7-trimethylen-3-amino-2-methyl-2H-1,4-benzoxazin Trihydrochlorid

Nach üblichen Methoden erhält man
6-(5-{[N-Isopropyl]-amino-n-pentyl}-aminomethyl)-3-amino-2-methyl-2H-1,4-benzoxazin Succinat [Stöchiometrie 1-1,5 fach]
Fp.: 119.4 °C
6-(5-{[N-Isopropyl]-amino-n-pentyl}-aminomethyl)-3-amino-2-methyl-2H-1,4-benzoxazin Trispropionat Fp.: 134.9 °C
6-(5-{[N-Isopropyl]-amino-n-pentyl}-aminomethyl)-3-amino-2-methyl-2H-1,4-benzoxazin Oxalat [Stöchiometrie 1-1,5 fach] Fp.: 215.2 °C

## Patentansprüche

1. Verbindungen der Formel I, deren tautomere und isomere Formen und Salze 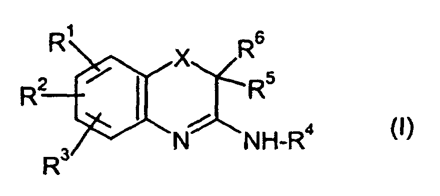 worin
X Sauerstoff oder Schwefel
R¹ -(CHR⁹)ₙ-NR⁷-A-NR⁸-B
R² Wasserstoff ist oder
R¹ und R² gemeinsam mit zwei benachbarten Kohlenstoffatomen einen 3-8gliedrigen Ring bilden, der mit -(CHR⁹)ᵣ-NR⁷-A-NR⁸-B substituiert ist, und mit C₁₋₄-Alkyl substituiert sein kann,
R³ Wasserstoff, Halogen, NO₂, Cyano, CF₃, -OCF₃, -S-R⁹, -O-R⁹, C₃₋₇-Cycloalkyl, -NR⁹-C(=NR¹⁰)-R¹¹, -NH-CS-NR¹²R¹³ , NH-CO-NR¹²R¹³, -SO₂NR¹²R¹³, -CO-NR¹²R¹³, -CO-R¹⁴, NR¹⁵R¹⁶, C₆₋₁₀Aryl, das gegebenenfalls mit Halogen, Cyano, C₁₋₄-Alkyl, -S-R⁹ oder -O-R⁹ substituiert ist,
5- oder 6gliedriges Heteroaryl mit 1 bis 4 Sauerstoff-, Schwefel- oder Stickstoffatomen
C₁₋₆-Alkyl, das gegebenenfalls mit Halogen, -OR⁹, -SR9, -NR¹²R¹³, =NR¹²,=NOC₁₋₆-Alkyl, =N-NHAryl, Phenyl, C₃₋₇-Cycloalkyl oder 5- oder 6gliedrigem Heteroaryl substituiert ist,
C₂₋₆-Alkenyl, das gegebenenfalls mit Halogen, CONH₂, C≡N oder Phenyl substituiert ist,
C₂₋₆-Alkinyl, das gegebenenfalls mit Halogen, CONH₂, C≡N oder Phenyl substituiert ist,
R⁴ Wasserstoff oder Acyl,
R⁵ Wasserstoff,
R⁶ C₁₋₆-Alkyl,
R⁷ Wasserstoff, C₁₋₆-Alkyl, das mit Phenyl substituiert sein kann, COOC₁₋₆-Alkyl oder CO-C₁₋₆-Alkyl,
R⁸ Wasserstoff, C₁₋₆-Alkyl, das mit Phenyl substiuiert sein kann, COOC₁₋₆-alkyl oder COC₁₋₆-Alkyl,
A geradkettiges oder verzweigtes C₁₋₆-Alkylen, geradkettiges oder verzweigtes C₁₋₆-Alkenylen oder -(CH₂)ₚ-Q-(CH₂)_{q}-,
B Wasserstoff oder -(CH₂)ₚ-U,
Q C₃₋₇-Cycloalkyl, Indanyl, 5-, 6- oder 7gliedriges gesättigtes Heterocycloalkyl mit 1 - 2 N-, O- oder S-Atomen, C₆₋₁₀-Aryl oder 5- oder 6gliedriges Heteroaryl mit 1 - 3 N-, O- oder S-Atomen, das mit Benzol anelliert sein kann,
U Wasserstoff, gegebenenfalls mit Halogen substituiertes C₁₋₆-Alkyl, C₃₋₇-Cycloalkyl, Indanyl, C₇₋₁₀-Bicycloalkyl, C₆₋₁₀-Aryl oder 5- oder 6gliedriges Heteroaryl mit 1 - 3 N-, O- oder S-Atomen, das mit Benzol anelliert sein kann, wobei der Aryl- und Heteroarylrest substituiert sein kann mit Halogen, C₁₋₄-Alkyl, C₁₋₄-Alkoxy, CF₃, NO₂, NH₂, N(C₁₋₄-Alkyl)₂, Cyano, CONH₂, -O-CH₂O-, -O-(CH₂)₂-O-, SO₂NH₂, OH, Phenoxy oder COOC₁₋₄-Alkyl,
oder
R⁸ und B gemeinsam mit dem Stickstoffatom einen 5 - 7gliedrigen gesättigten Heterocyclus bilden, der ein weiteres Sauerstoff-, Stickstoff- oder Schwefelatom enthalten kann und 1-3fach mit C₁₋₄-Alkyl oder einem gegebenenfalls mit Halogen substituierten Phenyl-, Benzyl- oder Benzoylrest substituiert sein kann oder einen ungesättigten 5gliedrigen Heterocyclus bilden, der 1 - 3 N-Atome enthalten und mit Phenyl, C₁₋₄-Alkyl oder Halogen substituiert sein kann, oder
R⁷ und A gemeinsam mit dem Stickstoffatom einen 5 - 7gliedrigen gesättigten Heterocyclus bildet, der ein weiteres Sauerstoff-, Stickstoff- oder Schwefelatom enhalten kann oder einen ungesättigten 5gliedrigen Heterocyclus bildet, der 1 - 3 N-Atome enthalten kann,
m 0, 1 oder 2,
n und r 0, 1 bis 6,
p und q 0 bis 6 bedeuten,
R⁹ und R¹⁰ Wasserstoff oder C₁₋₆-Alkyl,
R¹¹ C₁₋₆-Alkyl, -NH₂, -NH-CH₃,-NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6gliedriges Heteroaryl mit 1 bis 4 Stickstoff-, Schwefel- oder Sauerstoffatomen,
R¹² und R¹³ Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
R¹⁴ Wasserstoff, Hydroxy, C₁₋₆-Alkoxy, Phenyl, gegebenenfalls mit CO₂H, CO₂C₁₋₆-Alkyl, Hydroxy, C₁₋₄-Alkoxy, Halogen, NR¹⁵R¹⁶,CONR¹²R¹³ oder Phenyl substituiertes C₁₋₆-Alkyl oder gegebenenfalls mit Phenyl, Cyano, CONR¹²R¹³ oder CO₂C₁₋₄-Alkyl substituiertes C₂₋₆-Alkenyl,
R¹⁵ und R¹⁶ Wasserstoff, C₁₋₆-Alkyl, Phenyl oder Benzyl oder
R¹⁵, R¹⁶ gemeinsam mit dem Stickstoffatom einen gesättigten 5-, 6- oder 7gliedrigen Ring bilden, der ein weiteres Stickstoff-, Sauerstoff- oder Schwefelatom enthalten und substituiert sein kann mit C₁₋₄-Alkyl, Phenyl, Benzyl oder Benzoyl
bedeuten, wobei
falls X= O, R⁶ Methyl und R², R³, R⁴ und R⁵ Wasserstoff bedeuten, R¹ nicht 6-((4-Aminobenzyl)aminomethyl), 6-((4-Dimethylaminobenzyl)aminomethyl), 6-((4-Aminobenzyl)(tert.-butyloxycarbonyl)aminomethyl), 6-((4-Dimethylaminobenzyl) (tert.-butyloxycarbonyl)aminomethyl) ist.

2. Verbindungen gemäß Anspruch 1, worin R⁴ Wasserstoff ist.

3. Verbindungen nach einem der Ansprüche 1 - 2, worin R³ Wasserstoff ist.

4. Verbindungen nach einem der Ansprüche 1 - 3, worin R⁷ und R⁸ Wasserstoff ist.

5. Verbindungen nach einem der Ansprüche 1 - 4, worin R¹ und R² gemeinsam mit zwei benachbarten Kohlenstoffatomen einen 5 - 6gliedrigen Ring bedeutet, der mit -(CHR⁹)ᵣ-NR⁷-A-NR⁸B substituiert ist.

6. Verbindungen nach Anspruch 5, worin r = null ist.

7. Verbindungen gemäß einem der Ansprüche 1 - 6, worin A geradkettiges oder verzweigtes C₁₋₆-Alkylen oder -(CH₂)ₚ-Q-(CH₂)_{q}- und p und q 1 - 4 bedeuten.

8. Verbindungen nach Anspruch 1, worin der Heteroarylrest U Imidazol, Indol, Isooxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin, Chinolin, 2-C₁₋₆-Alkyl-3-amino-1,4-benzoxazin oder 2-C₁₋₆-Alkyl-3-keto-1,4-benzoxazin bedeutet.

9. Verbindungen gemäß einem der Ansprüche 1 - 7, worin U Wasserstoff, gegebenenfalls mit Halogen substituiertes C₁₋₆-Alkyl, C₃₋₇-Cycloalkyl oder gegebenenfalls mit Halogen, C₁₋₄-Alkyl, C₁₋₄-Alkoxy, CF₃, NO₂, NH₂, N(C₁₋₄-Alkyl)₂, Cyano, CONH₂, -O-CH₂-O-, -O-(CH₂)₂-O-, SO₂NH₂, OH, Phenoxy oder COOC₁₋₄-Alkyl substituiertes Phenyl bedeutet.

10. 6-((3-aminomethyl)-benzyl-aminomethyl)-3-amino-2-methyl-2H-1,4-benzoxazin Trihydrochlorid
6-(meta-(N-[3-Keto-2-methyl-2H-1,4-benzoxazin-6-yl]-methyl-aminomethyl-benzylaminomethyl)-3-amino-2-methyl-2H-1,4-benzoxazin Trihydrochlorid
6-(meta-(N-[3-Amino-2-methyl-2H-1,4-benzoxazin-6-yl]-methyl-aminomethyl-benzylaminomethyl)-3-amino-2-methyl-2H-1,4-benzoxazin Trihydrochlorid
6-((4-aminomethyl)-benzyl-aminomethyl)-3-amino-2-methyl-2H-1,4-benzoxazin Trihydrochlorid
6-(para-(N-[3-Amino-2-methyl-2H-1,4-benzoxazin-6-yl]-methyl-aminomethyl)-benzylaminomethyl)-3-amino-2-methyl-2H-1,4-benzoxazin Trihydrochlorid
6-(para-(N-[3-Keto-2-methyl-2H-1,4-benzoxazin-6-yl]-methyl-aminomethyl)-benzylaminomethyl)-3-amino-2-methyl-2H-1,4-benzoxazin Trihydrochlorid
6-((3-aminomethyl-cyclohex-1-yl)-methyl-aminomethyl)-3-amino-2-methyl-2H-1,4-benzoxazin Trihydrochlorid
6-(3-(N-[3-Amino-2-methyl-2H-1,4-benzoxazin-6-yl]-methyl-aminomethyl)-cyclohex-1-ylmethyl-aminomethyl)-3-amino-2-methyl-2H-1,4-benzoxazin Trihydrochlorid 6-((omega-aminobutyl-aminomethyl)-3-amino-2-methyl-2H-1,4-benzoxazin Trihydrochlorid
6-((omega-aminopentyl-aminomethyl)-3-amino-2-methyl-2H-1,4-benzoxazin Trihydrochlorid
6-((omega-aminohexyl-aminomethyl)-3-amino-2-methyl-2H-1,4-benzoxazin Trihydrochlorid
6-((3-[4-Nitrobenzyl]- aminomethyl)-benzylaminomethyl)-3-amino-2-methyl-2H-1,4-benzoxazin Trihydrochlorid
6-((3-[2-Methylbenzyl]- aminomethyl)-benzylaminomethyl)-3-amino-2-methyl-2H-1,4-benzoxazin Trihydrochlorid
6-((3-[2,4-Dichlorbenzyl]- aminomethyl)-benzylaminomethyl)-3-amino-2-methyl-2H-1,4-benzoxazin Trihydrochlorid
6-((3-[3-Chlorbenzyl]- aminomethyl)-benzylaminomethyl)-3-amino-2-methyl-2H-1,4-benzoxazin Trihydrochlorid
6-((3-[3,4-Dichlorbenzyl]- aminomethyl)-benzylaminomethyl)-3-amino-2-methyl-2H-1,4-benzoxazin Trihydrochlorid
6-((3-Benzylaminomethyl)-benzylaminomethyl)-3-amino-2-methyl-2H-1,4-benzoxazin Trihydrochlorid
gemäß Anspruch 1.

11. Arzneimittel enthaltend eine Verbindung gemäß Anspruch 1 - 10 und einen oder mehrere pharmazeutisch übliche Träger- oder Hilfsstoffe.

12. Verwendung einer Verbindung gemäß Anspruch 1 - 10 zur Herstellung eines Arzneimittels zur Behandlung einer Erkrankung, die durch NOS ausgelöst wird.

13. Verwendung gemäß Anspruch 12 zur Behandlung neurodegenerativer Erkrankungen.

14. Verfahren zur Herstellung einer Verbindung der Formel I nach Anspruch 1, **dadurch gekennzeichnet, daß** man eine Verbindung der Formel II oder deren Salz 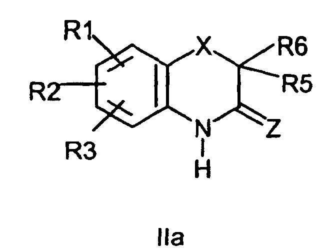 oder 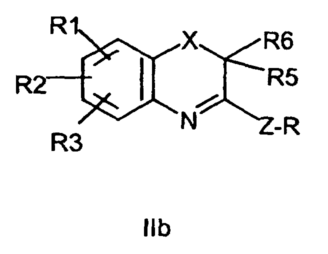 worin
R¹, R², R³, R⁵, R⁶ und X die obige Bedeutung haben, Z Sauerstoff oder Schwefel ist und R C₁₋₆-Alkyl bedeutet, mit Ammoniak oder primären Aminen umsetzt, wobei vorhandene Aminogruppen gegebenenfalls intermediär geschützt sind, und gewünschtenfalls anschließend acyliert, die Isomeren trennt oder die Salze bildet.

15. Verbindungen der Formel IIa und IIb 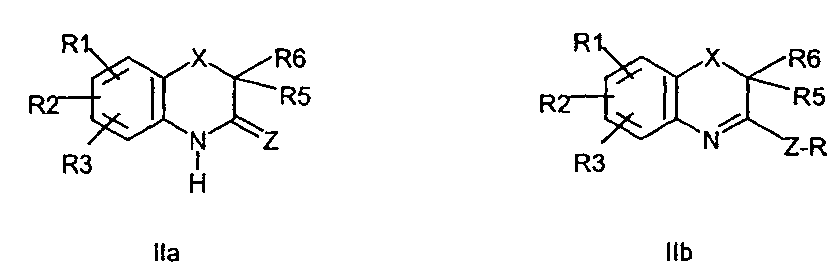 worin
R¹, R², R³, R⁵, R⁶ und X die die in Anspruch 1 genannte Bedeutung haben, Z Sauerstoff oder Schwefel ist und R C₁₋₆-Alkyl bedeutet.

16. Verbindungen nach einem der Ansprüche 1-9, worin R⁷ und A oder R⁸ und B gemeinsam mit dem Stickstoffatom unabhängig voneinander Imidazol, Pyrrol, Pyrazol oder Triazol bedeuten.

17. Verbindungen nach einem der Ansprüche 1-9, worin R⁷ und A oder R⁸ und B gemeinsam mit dem Stickstoffatom unabhängig voneinander einen 5-7gliedrigen gesättigten Heterocyclus bilden ausgewählt aus Piperidin, Pyrrolidin, Morpholin, Thiomorpholin, Hexahydroazepin, Piperazin, N-Methyl-piperazin, 2,6-Dimethylmorpholin, Phenylpiperazin oder 4-(4-Fluorbenzoyl)-piperidin.

## Claims

1. Compounds of Formula I, their tautomeric and isomeric forms and salts: 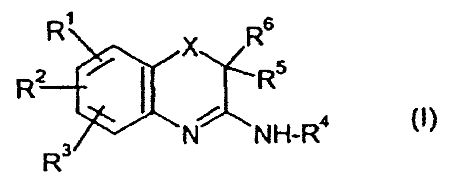 in which
X is oxygen or sulphur
R¹ is -(CHR⁹)ₙ-NR⁷-A-NR⁸-B
R² is hydrogen or
R¹ and R² together with two adjacent carbon atoms form a 3-8-membered ring which is substituted by -(CHR⁹)ᵣ-NR⁷-A-NR⁸-B and may be substituted by C₁₋₄-alkyl,
R³ is hydrogen, halogen, NO₂, cyano, CF₃, -OCF₃, --S-R⁹, -O-R⁹, C₃₋₇-cycloalkyl, -NR⁹-C(=NR¹⁰)-R¹¹, -NH-CS-NR¹²R¹³, NH-CO-NR¹²R¹³, -SO₂NR¹²R¹³, -CO-NR¹²R¹³, -CO-R¹⁴, NR¹⁵R¹⁶, C₆₋₁₀aryl which is optionally substituted with halogen, cyano, C₁₋₄-alkyl, -S-R⁹ or -O-R⁹,
5- or 6-membered heteroaryl having 1 to 4 oxygen, sulphur or nitrogen atoms
C₁₋₆-alkyl which is optionally substituted by halogen, -OR⁹, -SR⁹, -NR¹²R¹³, =NR¹², =NOC₁₋₆-alkyl, =N-NHaryl, phenyl, C₃₋₇-cycloalkyl or 5- or 6-membered heteroaryl,
C₂₋₆-alkenyl which is optionally substituted by halogen, CONH₂, C≡N or phenyl,
C₂₋₆-alkynyl which is optionally substituted by halogen, CONH₂, C≡N or phenyl,
R⁴ is hydrogen or acyl,
R⁵ is hydrogen,
R⁶ is C₁₋₆-alkyl,
R⁷ is hydrogen, C₁₋₆-alkyl which may be substituted by phenyl, or COOC₁₋₆-alkyl or CO-C₁₋₆-alkyl,
R⁸ is hydrogen, C₁₋₆-alkyl which may be substituted by phenyl, or COOC₁₋₆-alkyl or COC₁₋₆-alkyl,
A is straight-chain or branched C₁₋₆-alkylene, straight-chain or branched C₁₋₆-alkenylene or -(CH₂)ₚ-Q-(CH₂)_{q}-,
B is hydrogen or -(CH₂)ₚ-U,
Q is C₃₋₇-cycloalkyl, indanyl, 5-, 6- or 7-membered saturated heterocycloalkyl having 1-2 N, O or S atoms, C₆₋₁₀-aryl or 5- or 6-membered heteroaryl having 1-3 N, O or S atoms, which may be fused to benzene,
U is hydrogen, optionally halogen-substituted C₁₋₆-alkyl, C₃₋₇-cycloalkyl, indanyl, C₇₋₁₀-bicycloalkyl, C₆₋₁₀-aryl or 5- or 6-membered heteroaryl having 1-3 N, O or S atoms, which may be fused to benzene, where the aryl and heteroaryl radicals may be substituted by halogen, C₁₋₄-alkyl, C₁₋₄-alkoxy, CF₃, NO₂, NH₂, N(C₁₋₄-alkyl)₂, cyano, CONH₂, -O-CH₂-O-,
-O-(CH₂)₂-O-, SO₂NH₂, OH, phenoxy or COOC₁₋₄-alkyl,
or
R⁸ and B together with the nitrogen atom form a 5-7-membered saturated heterocycle which may contain a further oxygen, nitrogen or sulphur atom and may be substituted 1-3 times by C₁₋₄-alkyl or an optionally halogen-substituted phenyl, benzyl or benzoyl radical, or form an unsaturated 5-membered heterocycle which may contain 1-3 N atoms and be substituted by phenyl, C₁₋₄-alkyl or halogen, or
R⁷ and A together with the nitrogen atom forms a 5-7-membered saturated heterocycle which may contain a further oxygen, nitrogen or sulphur atom, or forms an unsaturated 5-membered heterocycle which may contain 1-3 N atoms,
m is 0, 1 or 2,
n and r are 0, 1 to 6,
p and q are 0 to 6,
R⁹ and R¹⁰ are hydrogen or C₁₋₆-alkyl,
R¹¹ is C₁₋₆-alkyl, -NH₂, -NH-CH₃, -NH-CN, optionally halogen-, C₁₋₄-alkyl- or CF₃-substituted C₆₋₁₀-aryl or optionally halogen-, C₁₋₄-alkyl- or CF₃-substituted 5- or 6-membered heteroaryl having 1 to 4 nitrogen, sulphur or oxygen atoms,
R¹² and R¹³ are hydrogen, C₁₋₆-alkyl, optionally halogen- or C₁₋₄-alkyl-substituted phenyl, optionally halogen- or C₁₋₄-alkyl-substituted benzyl or C₃₋₇-cycloalkyl,
R¹⁴ is hydrogen, hydroxyl, C₁₋₆-alkoxy, phenyl, optionally CO₂H-, CO₂C₁₋₆-alkyl-, hydroxyl-, C₁₋₄-alkoxy-, halogen-, NR¹⁵R¹⁶-, CONR¹²R¹³- or phenyl-substituted C₁₋₆-alkyl or optionally phenyl-, cyano-, CONR¹²R¹³- or CO₂C₁₋₄-alkyl-substituted C₂₋₆-alkenyl,
R¹⁵ and R¹⁶ are hydrogen, C₁₋₆-alkyl, phenyl or benzyl or
R¹⁵, R¹⁶ together with the nitrogen atom form a saturated 5-, 6- or 7-membered ring which may contain a further nitrogen, oxygen or sulphur atom and be substituted by C₁₋₄-alkyl, phenyl, benzyl or benzoyl,
where
if X is O, R⁶ is methyl and R², R³, R⁴ and R⁵ are hydrogen, R¹ is not 6-((4-aminobenzyl)aminomethyl), 6-((4-dimethylaminobenzyl)aminomethyl), 6-((4-aminobenzyl)(tert-butyloxycarbonyl)aminomethyl), 6-((4-dimethylaminobenzyl)(tert-butyloxycarbonyl)aminomethyl).

2. Compounds according to Claim 1, in which R⁴ is hydrogen.

3. Compounds according to either of Claims 1-2, in which R³ is hydrogen.

4. Compounds according to any of Claims 1-3, in which R⁷ and R⁸ is hydrogen.

5. Compounds according to any of Claims 1-4, in which R¹ and R² together with two adjacent carbon atoms is a 5-6-membered ring which is substituted by -(CHR⁹)ᵣ-NR⁷-A-NR⁸B.

6. Compounds according to Claim 5, in which r is zero.

7. Compounds according to any of Claims 1-6, in which A is straight-chain or branched C₁₋₆-alkylene or -(CH₂)ₚ-Q-(CH₂)_{q}- and p and q are 1-4.

8. Compounds according to Claim 1, in which the heteroaryl radical U is imidazole, indole, isooxazole, isothiazole, furan, oxadiazole, oxazole, pyrazine, pyridazine, pyrimidine, pyridine, pyrazole, pyrrole, tetrazole, thiazole, triazole, thiophene, thiadiazole, benzimidazole, benzofuran, benzoxazole, isoquinoline, quinoline, 2-C₁₋₆-alkyl-3-amino-1,4-benzoxazine or 2-C₁₋₆-alkyl-3-keto-1,4-benzoxazine.

9. Compounds according to any of Claims 1-7, in which U is hydrogen, optionally halogen-substituted C₁₋₆-alkyl, C₃₋₇-cycloalkyl or phenyl which is optionally substituted by halogen, C₁₋₄-alkyl, C₁₋₄-alkoxy, CF₃, NO₂, NH₂, N(C₁₋₄-alkyl)₂, cyano, CONH₂, -O-CH₂-O-, -O-(CH₂)₂-O-, SO₂NH₂, OH, phenoxy or COOC₁₋₄-alkyl.

10. 6-((3-Aminomethyl)benzylaminomethyl)-3-amino-2-methyl-2H-1,4-benzoxazine trihydrochloride
6-(meta-(N-[3-Keto-2-methyl-2H-1,4-benzoxazin-6-yl]-methylaminomethylbenzylaminomethyl)-3-amino-2-methyl-2H-1,4-benzoxazine trihydrochloride
6-(meta-(N-[3-Amino-2-methyl-2H-1,4-benzoxazin-6-yl]-methylaminomethylbenzylaminomethyl)-3-amino-2-methyl-2H-1,4-benzoxazine trihydrochloride
6-((4-Aminomethyl)benzylaminomethyl)-3-amino-2-methyl-2H-1,4-benzoxazine trihydrochloride
6-(para-(N-[3-Amino-2-methyl-2H-1,4-benzoxazin-6-yl]-methylaminomethyl)benzylaminomethyl)-3-amino-2-methyl-2H-1,4-benzoxazine trihydrochloride
6-(para-(N-[3-Keto-2-methyl-2H-1,4-benzoxazin-6-yl]-methylaminomethyl)benzylaminomethyl)-3-amino-2-methyl-2H-1,4-benzoxazine trihydrochloride
6-((3-aminomethylcyclohex-1-yl)methylaminomethyl)-3-amino-2-methyl-2H-1,4-benzoxazine trihydrochloride
6-(3-(N-[3-Amino-2-methyl-2H-1,4-benzoxazin-6-yl]-methylaminomethyl)cyclohex-1-ylmethylaminomethyl)-3-amino-2-methyl-2H-1,4-benzoxazine trihydrochloride
6-((omega-aminobutylaminomethyl)-3-amino-2-methyl-2H-1,4-benzoxazine trihydrochloride
6-((omega-aminopentylaminomethyl)-3-amino-2-methyl-2H-1,4-benzoxazine trihydrochloride
6-((omega-aminohexylaminomethyl)-3-amino-2-methyl-2H-1,4-benzoxazine trihydrochloride
6-((3-[4-Nitrobenzyl]aminomethyl)benzylaminomethyl)-3-amino-2-methyl-2H-1,4-benzoxazine trihydrochloride
6-((3-[2-Methylbenzyl]aminomethyl)benzylaminomethyl)-3-amino-2-methyl-2H-1,4-benzoxazine trihydrochloride
6-((3-[2,4-Dichlorobenzyl]aminomethyl)benzylaminomethyl)-3-amino-2-methyl-2H-1,4-benzoxazine trihydrochloride
6-((3-[3-Chlorobenzyl]aminomethyl)benzylaminomethyl)-3-amino-2-methyl-2H-1,4-benzoxazine trihydrochloride
6-((3-[3,4-Dichlorobenzyl]aminomethyl)benzylaminomethyl)-3-amino-2-methyl-2H-1,4-benzoxazine trihydrochloride
6-((3-Benzylaminomethyl)benzylaminomethyl)-3-amino-2-methyl-2H-1,4-benzoxazine trihydrochloride
according to Claim 1.

11. Pharmaceutical composition which comprises a compound according to Claim 1-10 and one or more pharmaceutically customary vehicles or adjuvants.

12. Use of a compound according to Claim 1-10 for producing a pharmaceutical composition for treating a disease caused by NOS.

13. Use according to Claim 12 for treating neurodegenerative diseases.

14. Process for the production of a compound of Formula I according to Claim 1, **characterized in that** a compound of Formula II or its salt 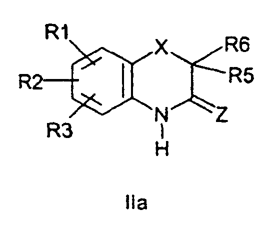 or 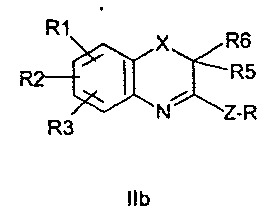 in which
R¹, R², R³, R⁵, R⁶ and X have the abovementioned meaning, Z is oxygen or sulphur and R means C₁₋₆ alkyl, is reacted with ammonia or primary amines, existing amino groups being optionally intermediately protected, and if desired is then acylated, the isomers are separated or the salts are formed.

15. Compounds of Formula IIa and IIb 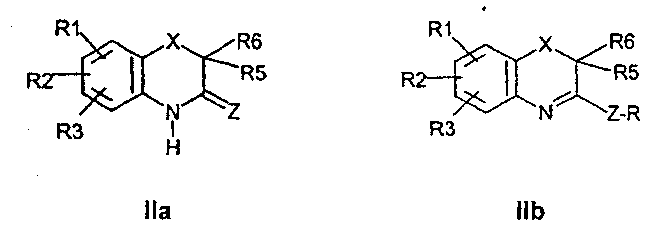 in which
R¹, R², R³, R⁵, R⁶ and X have the meaning given in Claim 1, Z means oxygen or sulphur and R means C₁₋₆ alkyl.

16. Compounds according to any of Claims 1-9, in which R⁷ and A or R⁸ and B together with the nitrogen atom are independently of one another imidazole, pyrrole, pyrazole or triazole.

17. Compounds according to any of Claims 1-9, in which R⁷ and A or R⁸ and B together with the nitrogen atom form independently of one another a 5-7-membered saturated heterocycle selected from piperidine, pyrrolidine, morpholine, thiomorpholine, hexahydroazepine, piperazine, N-methylpiperazine, 2,6-dimethylmorpholine, phenylpiperazine or 4-(4-fluorobenzoyl)-piperidine.

## Revendications

1. Composés de formule I, leurs formes tautomères et isomères et sels 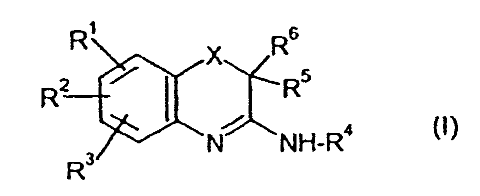 dans laquelle
X représente un oxygène ou un soufre
R¹ représente -(CHR⁹)ₙ-NR⁷-A-NR⁸-B
R² représente un hydrogène, ou
R¹ et R², conjointement avec deux atomes de carbone adjacents, forment un noyau à 3 - 8 chaînons, qui est substitué par -(CHR⁹)ᵣ-NR⁷-A-NR⁸-B et peut être substitué par un alkyle en C₁₋₄,
R³ représente un hydrogène, un halogène, NO₂, un cyano, CF₃, -OCF₃, -S-R⁹, -O-R⁹, un cycloalkyle en C₃₋₇, -NR⁹-C(=NR¹⁰)-R¹¹, -NH-CS-NR¹²R¹³, NH-CO-NR¹²R¹³, -SO₂NR¹²R¹³, -CO-NR¹²R¹³, -CO-R¹⁴, NR¹⁵R¹⁶, un aryle en C₆₋₁₀, qui est éventuellement substitué par un halogène, un cyano, un alkyle en C₁₋₄, -S-R⁹ ou -O-R⁹,
un hétéroaryle à 5 ou 6 chaînons ayant de 1 à 4 atomes d'oxygène, de soufre ou d'azote,
un alkyle en C₁₋₆ qui est éventuellement substitué par un halogène, -OR⁹, -SR⁹, -NR¹²R¹³, =NR¹², un =NOC₁₋₆-alkyle, un =N-NHaryle, un phényle, un cycloalkyle en C₃₋₇ ou un hétéroaryle à 5 ou 6 chaînons,
un alcényle en C₂₋₆ qui est éventuellement substitué par un halogène, CONH₂, C≡N ou un phényle,
un alcynyle en C₂₋₆, qui est éventuellement substitué par un halogène, CONH₂, C≡N ou un phényle,
R⁴ représente un hydrogène ou un acyle,
R⁵ représente un hydrogène,
R⁶ représente un alkyle en C₁₋₆,
R⁷ représente un hydrogène, un alkyle en C₁₋₆ qui peut être substitué par un phényle, un COOC₁₋₆-alkyle ou un CO-C₁₋₆-alkyle,
R⁸ représente un hydrogène, un alkyle en C₁₋₆ qui peut être substitué par un phényle, un COOC₁₋₆-alkyle ou un COC₁₋₆-alkyle,
A représente un alkylène en C₁₋₆ linéaire ou ramifié, un alcénylène en C₁₋₆ linéaire ou ramifié ou -(CH₂)ₚ-Q-(CH₂)_{q}-,
B représente un hydrogène ou -(CH₂)ₚ-U,
Q représente un cycloalkyle en C₃₋₇, un indanyle, un hétérocycloalkyle saturé à 5, 6 ou 7 chaînons ayant 1 - 2 atomes d'azote, d'oxygène ou de soufre, un aryle en C₆₋₁₀ ou un hétéroaryle à 5 ou 6 chaînons ayant 1 - 3 atomes d'azote, d'oxygène ou de soufre, qui peut être condensé sur un benzène,
U représente un hydrogène, un alkyle en C₁₋₆ éventuellement substitué par un halogène, un cycloalkyle en C₃₋₇, un indanyle, un bicycloalkyle en C₇₋₁₀, un aryle en C₆₋₁₀ ou un hétéroaryle à 5 ou 6 chaînons ayant 1 - 3 atomes d'azote, d'oxygène ou de soufre, qui peut condensé sur un benzène, où les radicaux aryle et hétéroaryle peuvent être substitués par un halogène, un alkyle en C₁₋₄, un alcoxy en C₁₋₄, CF₃, NO₂, NH₂, un N(C₁₋₄-alkyle)₂, un cyano, CONH₂, -O-CH₂-O-, -O-(CH₂)₂-O-, SO₂NH₂, OH, un phénoxy ou un COOC₁₋₄-alkyle,
ou
R⁸ et B, conjointement avec l'atome d'azote, forment un hétérocycle saturé à 5 - 7 chaînons, qui peut renfermer un atome d'oxygène, d'azote ou de soufre supplémentaire et peut être mono- à tri-substitué par un alkyle en C₁₋₄ ou un radical phényle, benzyle ou benzoyle éventuellement substitué par' un halogène, ou forment un hétérocycle insaturé à 5 chaînons, qui peut renfermer 1 - 3 atomes d'azote et peut être substitué par un phényle, un alkyle en C₁₋₄ ou un halogène, ou
R⁷ et A, conjointement avec l'atome d'azote, forment un hétérocycle saturé à 5 - 7 chaînons, qui peut renfermer un atome d'oxygène, d'azote ou de soufre supplémentaire, ou forment un hétérocycle insaturé à 5 chaînons, qui peut renfermer 1 - 3 atomes d'azote,
m vaut 0, 1 ou 2,
n et r valent 0, de 1 à 6,
p et q valent de 0 à 6,
R⁹ et R¹⁰ représentent un hydrogène ou un alkyle en C₁₋₆,
R¹¹ représente un alkyle en C₁₋₆, -NH₂, -NH-CH₃, -NH-CN, un aryle en C₆₋₁₀ éventuellement substitué par un halogène, un alkyle en C₁₋₄ ou CF₃, ou un hétéroaryle à 5 ou 6 chaînons, éventuellement substitué par un halogène, un alkyle en C₁₋₄ ou CF₃ et ayant de 1 à 4 atomes d'azote, de soufre ou d'oxygène,
R¹² et R¹³ représentent un hydrogène, un alkyle en C₁₋₆, un phényle éventuellement substitué par un halogène ou un alkyle en C₁₋₄, un benzyle éventuellement substitué par un halogène ou un alkyle en C₁₋₄, ou un cycloalkyle en C₃₋₇,
R¹⁴ représente un hydrogène, un hydroxy, un alcoxy en C₁₋₆, un phényle, un alkyle en C₁₋₆ éventuellement substitué par CO₂H, un CO₂C₁₋₆-alkyle, un hydroxy, un alcoxy en C₁₋₄, un halogène, NR¹⁵R¹⁶, CONR¹²R¹³ ou un phényle, ou un alcényle en C₂₋₆ éventuellement substitué par un phényle, un cyano, CONR¹²R¹³ ou un CO₂C₁₋₄-alkyle,
R¹⁵ et R¹⁶ représentent un hydrogène, un alkyle en C₁₋₆, un phényle ou un benzyle, ou
R¹⁵ et R¹⁶, conjointement avec l'atome d'azote, forment un noyau saturé à 5, 6 ou 7 chaînons, qui peut renfermer un atome d'azote, d'oxygène ou de soufre supplémentaire et peut être substitué par un alkyle en C₁₋₄, un phényle, un benzyle ou un benzoyle,
où
si X = O, R⁶ représente un méthyle et R², R³, R⁴ et R⁵ représentent un hydrogène, R¹ ne représente pas un 6-((4-aminobenzyl)aminométhyle), un 6-((4-diméthylaminobenzyl)aminométhyle), un 6-((4-aminobenzyl)(tert-butyloxycarbonyl)aminométhyle), un 6-((4-diméthylaminobenzyl)(tert-butyloxycarbonyl)aminométhyle).

2. Composés selon la revendication 1, dans lesquels R⁴ représente un hydrogène.

3. Composés selon l'une quelconque des revendications 1 et 2, dans lesquels R³ représente un hydrogène.

4. Composés selon l'une quelconque des revendications 1 à 3, dans lesquels R⁷ et R⁸ représentent un hydrogène.

5. Composés selon l'une quelconque des revendications 1 à 4, dans lesquels R¹ et R², conjointement avec deux atomes de carbone adjacents, représentent un noyau à 5 - 6 chaînons qui est substitué par (CHR⁹)ᵣ-NR⁷-A-NR⁸B.

6. Composés selon la revendication 5, dans lesquels r vaut zéro.

7. Composés selon l'une quelconque des revendications 1 à 6, dans lesquels A représente un alkylène en C₁₋₆ linéaire ou ramifié ou -(CH₂)ₚ-Q-(CH₂)_{q}- et p et q valent de 1 à 4.

8. Composés selon la revendication 1, dans lesquels le radical hétéroaryle U représente un imidazole, un indole, un iso-oxazole, un isothiazole, un furane, un oxadiazole, un oxazole, une pyrazine, une pyridazine, une pyrimidine, une pyridine, un pyrazole, un pyrrole, un tétrazole, un thiazole, un triazole, un thiophène, un thiadiazole, un benzimidazole, un benzofurane, un benzoxazole, une isoquinoléine, une quinoléine, une 2-C₁₋₆-alkyl-3-amino-1,4-benzoxazine ou une 2-C₁₋₆-alkyl-3-céto-1,4-benzoxazine.

9. Composés selon l'une quelconque des revendications 1 à 7, dans lesquels U représente un hydrogène, un alkyle en C₁₋₆ éventuellement substitué par un halogène, un cycloalkyle en C₃₋₇ ou un phényle éventuellement substitué par un halogène, un alkyle en C₁₋₄, un alcoxy en C₁₋₄, CF₃, NO₂, NH₂, un N(C₁₋₄-alkyle)₂, un cyano, CONH₂, -O-CH₂-O-, -O-(CH₂)₂-O-, SO₂NH₂, OH, un phénoxy ou un COOC₁₋₄-alkyle.

10. Trichlorhydrate de 6-((3-aminométhyl)benzylaminométhyl)-3-amino-2-méthyl-2H-1,4-benzoxazine
Trichlorhydrate de 6-(méta-(N-[3-céto-2-méthyl-2H-1,4-benzoxazin-6-yl]méthylaminométhylbenzylaminométhyl)-3-amino-2-méthyl-2H-1,4-benzoxazine
Trichlorhydrate de 6-(méta-(N-[3-amino-2-méthyl-2H-1,4-benzoxazin-6-yl]méthylaminométhylbenzylaminométhyl)-3-amino-2-méthyl-2H-1,4-benzoxazine
Trichlorhydrate de 6-((4-aminométhyl)benzylaminométhyl)-3-amino-2-méthyl-2H-1,4-benzoxazine
Trichlorhydrate de 6-(para-(N-[3-amino-2-méthyl-2H-1,4-benzoxazin-6-yl]méthylaminométhyl)benzylaminométhyl)-3-amino-2-méthyl-2H-1,4-benzoxazine
Trichlorhydrate de 6-(para-(N-[3-céto-2-méthyl-2H-1,4-benzoxazin-6-yl]méthylaminométhyl)benzylaminométhyl)-3-amino-2-méthyl-2H-1,4-benzoxazine
Trichlorhydrate de 6-((3-aminométhylcyclohex-1-yl)-méthylaminométhyl)-3-amino-2-méthyl-2H-1,4-benzoxazine
Trichlorhydrate de 6-(3-(N-[3-amino-2-méthyl-2H-1,4-benzoxazin-6-yl]méthylaminométhyl)cyclohex-1-ylméthylaminométhyl)-3-amino-2-méthyl-2H-1,4-benzoxazine
Trichlorhydrate de 6-((oméga-aminobutylaminométhyl)-3-amino-2-méthyl-2H-1,4-benzoxazine
Trichlorhydrate de 6-((oméga-aminopentylaminométhyl)-3-amino-2-méthyl-2H-1,4-benzoxazine
Trichlorhydrate de 6-((oméga-aminohexylaminométhyl)-3-amino-2-méthyl-2H-1,4-benzoxazine
Trichlorhydrate de 6-((3-[4-nitrobenzyl]aminométhyl)-benzylaminométhyl)-3-amino-2-méthyl-2H-1,4-benzoxazine
Trichlorhydrate de 6-((3-[2-méthylbenzyl]aminométhyl)-benzylaminométhyl)-3-amino-2-méthyl-2H-1,4-benzoxazine
Trichlorhydrate de 6-((3-[2,4-dichlorobenzyl]aminométhyl)benzylaminométhyl)-3-amino-2-méthyl-2H-1,4-benzoxazine
Trichlorhydrate de 6-((3-[3-chlorobenzyl]aminométhyl)-benzylaminométhyl)-3-amino-2-méthyl-2H-1,4-benzoxazine
Trichlorhydrate de 6-((3-[3,4-dichlorobenzyl]aminométhyl)benzylaminométhyl)-3-amino-2-méthyl-2H-1,4-benzoxazine
Trichlorhydrate de 6-((3-benzylaminométhyl)benzylaminométhyl)-3-amino-2-méthyl-2H-1,4-benzoxazine
selon la revendication 1.

11. Produit pharmaceutique contenant un composé selon les revendications 1 à 10 et un ou plusieurs supports ou auxiliaires habituels dans le domaine pharmaceutique.

12. Utilisation d'un composé selon les revendications 1 à 10 pour la préparation d'un produit pharmaceutique destiné au traitement d'une maladie causée par NOS.

13. Utilisation selon la revendication 12 pour le traitement de maladies neurodégénératives.

14. Procédé de préparation d'un composé de formule I selon la revendication 1, **caractérisé en ce qu'**un composé de formule II ou son sel 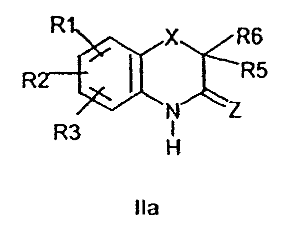 ou 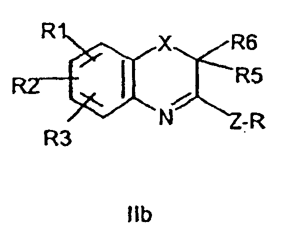 dans laquelle
R¹, R², R³, R⁵, R⁶ et X présentent la signification ci-dessus, Z représente un oxygène ou un soufre et R représente un alkyle en C₁₋₆, est mis à réagir avec de l'ammoniac ou des amines primaires, où les groupes amino présents sont éventuellement protégés de façon intermédiaire, et on procède ensuite, si on le souhaite, à une acylation, les isomères sont séparés ou les sels sont formés.

15. Composés de formules IIa et IIb 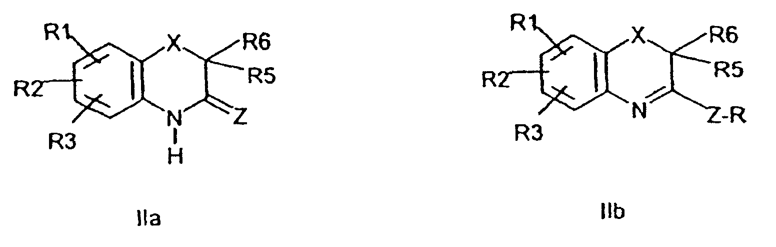 dans lesquelles
R¹, R², R³, R⁵, R⁶ et X présentent la signification mentionnée dans la revendication 1, Z représente un oxygène ou un soufre et R représente un alkyle en C₁₋₆.

16. Composés selon l'une quelconque des revendications 1 à 9, dans lesquels R⁷ et A ou R⁸ et B, conjointement avec l'atome d'azote, représentent, indépendamment les uns des autres, un imidazole, un pyrrole, un pyrazole ou un triazole.

17. Composés selon l'une quelconque des revendications 1 à 9, dans lesquels R⁷ et A ou R⁸ et B, conjointement avec l'atome d'azote, forment, indépendamment les uns des autres, un hétérocycle saturé à 5 - 7 chaînons, choisi parmi la pipéridine, la pyrrolidine, la morpholine, la thiomorpholine, l'hexahydroazépine, la pipérazine, la N-méthylpipérazine, la 2,6-diméthylmorpholine, la phénylpipérazine ou la 4-(4-fluorobenzoyl)pipéridine.
